# EUROPEAN PATENT APPLICATION

(11) **EP 4 763 967 A1**
(43) Date of publication of application: **24.06.2026**
(21) Application number: 24222593.6
(22) Date of filing: 20.12.2024
(51) Int. Cl.: C12M 1/32, C12M 1/00, C12M 1/34

(54) **A DEVICE FOR CULTIVATING 3D-CELL CLUSTER**

(71) Applicant: Koninklijke Philips N.V., 5656 AE Eindhoven (NL)
(72) Inventor: BECKERS, Lucas Johannes Anna Maria, Eindhoven (NL); VULDERS, Roland Cornelis Martinus, Eindhoven (NL)
(74) Representative: Philips Intellectual Property & Standards

(57) **Abstract**

Disclosed is a device for culturing a three-dimensional cell cluster in at least one recess having a recess depth of at least substantially 50 µm, an effective recess diameter of at least substantially 50 µm, and a recess opening area of at least substantially 0.002 mm²

## Description

### FIELD OF THE INVENTION

The invention relates to devices, systems, and methods of using the same, for cultivation of and/or experimentation with three-dimensional (3D) cell clusters such as e.g. spheroids and organoids.

### BACKGROUND OF THE INVENTION

In vitro grown or cultivated three-dimensional (3D) cell clusters, also referred to as 3D-cell cultures, such as in particular spheroids and organoids, have recently gained popularity for drug cytotoxicity evaluation and other biological and disease investigations to their ability to better mimic the complexity of in vivo systems co compared to the traditionally used two-dimensional (2D) cell clusters.

A 3D-cell cluster includes cells grown in all directions and as such is a better model to study cell characteristics such as for example cell-cell and cell-matrix interactions, transport dynamics for nutrients and drugs, migration, differentiation, polarization, gene expression, survival and growth of cells. The third dimension also provides more contact space for mechanical inputs and for cell adhesion, necessary for integrin ligation, cell contraction, intracellular signaling, solute diffusion and binding to effector proteins (like growth factors and enzymes). Furthermore, 3D-cell clusters have greater stability and longer lifespan than their 2D counterparts. For example, spheroids have been cultured for as long as 300 days while maintaining healthy, non-cancerous growth. The 3D-cell clusters are thus more suitable for longer-term studies, such as for example for demonstrating long-term effects of a drug or cancer growth.

Despite the growing interest, the study of 3D-cell clusters is accompanied by several challenges. One challenge is concerned with the commercialization of mature and user-friendly (microfluidic) devices that can be used for these studies, especially when it concerns high-throughput investigations or longer-term investigations. High-density well plates for growing of 3D-cell clusters have recently become available in the form of 384 and 1536-well plates of Greiner Bio-one or Corning Life Sciences. However, both types utilize a low attachment surface coating to create the 3D-cell clusters and this causes difficulties with performing investigation protocols.

Oftentimes investigation protocols require changes of fluidic culture medium wherein the 3D-cell clusters reside via flow or batch wise (pipetting) manipulation. This may be difficult when the 3D-cell clusters are not adhered to their culturing support (e.g. surface coating) as the manipulation can cause displacement or even removal of the 3D-cell clusters in the process. The displacement can cause further problems during relocating of the 3D-cell clusters in an analysis phase of (e.g. optical) investigations.

There is a need for improved devices, systems and methods for 3D-cell cluster experimentation and investigation.

### SUMMARY OF THE INVENTION

The invention is defined by the claims.

The disclosure provides for devices, systems, methods and uses that make use of small volume recesses, (also referred to herein as microwells) with dimensions in the order of those of small three-dimensional (3D) cell clusters such as in particular spheroids or organoids. The recesses are configured to be used for culturing such 3D-cell clusters therein. Typically, the recesses or microwells have dimensions a few millimeters or less. Such dimensions provide sufficient room for growth and cultivation of typically sized 3D-cell clusters in the recesses while confining them in a small space or volume.

This may have one or more of the following exemplary advantages. In some embodiments there may be improved cultivation of cells to form the 3D-cell clusters in both low-adhesion and specially designed increased adhesion surface devices. In some embodiments the size of 3D-cell clusters may be controlled with recess dimensions. In some embodiments improved experimentation is provided due to improved containment of 3D-cell clusters in a same location within the device, e.g. during manipulation of fluid for cultivation or other purposes. For example, the cultivation of 3D-cell clusters over longer periods of time may require their intermittent or even continuous perfusion with nutrients and this may be advantageously done with devices having channels and/or chambers as defined herein. Additionally, or alternatively, the (re)visiting for analysis of 3D-cell clusters during repeated analysis over time with other steps performed in between, may be improved due to the improved containment at a particular location in the device. Integration of several of the recesses in one chamber of a device, such as of a (standard) multi-well plate, may allow statistical analysis under the same or similar conditions for several 3D-cell clusters.

According to a first aspect there is provided a device for culturing a three-dimensional cell cluster. The device comprises: a substrate comprising a first substrate surface having at least one recess therein, the at least one recess being configured to hold the three-dimensional cell cluster at least partially therein. The at least one recess has a recess bottom and a recess depth of at least substantially 50 µm measured from the first substrate surface to the recess bottom. The at least one recess has a recess opening extending within the first substrate surface, the recess opening having an effective recess diameter of at least substantially 50 µm.

The recess depth preferably is at least substantially half of the effective recess diameter. The recess opening area may be at most substantially 20 mm².

The at least one recess preferably has a circularly shaped cross-section perpendicular to the direction of recess depth measurement. Preferably, the recess has a shape in the form of a cylinder or frustum of a cone the larger base of which forms the recess opening.

The recess bottom can have a concave shape.

In some preferred embodiments the recess bottom is permeable for fluid. Preferably, the permeability is implemented by a plurality of pores each of which having a pore diameter of at most substantially 20 µm. Preferably the pore diameter is at most 10 µm. The pore diameter can be in the range of 2 µm to 8 µm.

The bottom of the at least one recess may comprise a membrane having a membrane thickness of at most substantially 100 µm. In some embodiments the membrane has a thickness of at most substantially 50 µm. Such thickness allows pores to be conveniently made with laser ablation. The membrane thickness may be at least substantially 10 µm for integrity and manufacturability purposes. Preferably any of the plurality of holes defined hereinabove is then comprised in the membrane. The membrane preferably is comprised in the recess bottom.

In a preferred device the at least one recess comprises a plurality of recesses spaced apart across the surface. The plurality of recesses may be arranged in a one dimensional or a two-dimensional array of recesses. Such arrays may have one or more rows and columns of recesses. Such rows and columns of a two-dimensional array may be arranged at right or oblique angles.

A substrate of any of the devices defined herein preferably comprises an elastomeric material and the at least one recess is formed in the elastomeric material. Preferably the membrane, if present, is also formed in the elastomeric material. Preferably the elastomeric material is transparent for UV and/or visible light. Preferably the elastomeric material is a silicone-based elastomeric material such as e.g. PDMS.

Any of the devices as defined hereinbefore preferably comprises at least one chamber for holding a fluidic medium, the at least one chamber comprising the first substrate surface. In preferred embodiments, the at least one chamber comprise a chamber bottom and the first substrate surface is comprised in the chamber bottom The recesses are preferably located in the bottom of the at least one chamber.

A device as defined herein may further comprise a body made of a relatively stiff material, the body comprising the at least one chamber. In some embodiments the substrate may be made of the same relatively stiff material. In some embodiments the body may be an integrally formed body comprising the substrate. In some embodiments the body and substrate may separate parts in the device. In such embodiments preferably the body is made of a relatively stiff material and the substrate is made of an elastomeric material.

In some embodiments the at least one chamber comprises a plurality of chambers. Thus, each of the plurality of chambers is configured as defined for the at least one chamber and each one of the plurality of chamber thus comprises a first surface having the at least one recess.

In some embodiments the device comprises at least one fluid channel arranged to transport a fluidic medium from or to the at least one recess. The at least one fluid channel may be comprised in the substrate. Alternatively, or additionally, if a body separate from the substrate is comprised in the device, the at least one channel may be entirely or partially comprised in the body.

In preferred embodiments of devices as disclosed herein the device comprise the at least one chamber as defined herein and the at least one fluidic channel is fluidically connected to the at least one chamber. In some embodiments wherein the at least one chamber comprises a plurality of chambers the at least one fluid channel is fluidically connected to at least two of the plurality of chambers. In such embodiments at least one set of the plurality of chambers may be fluidically connected in parallel. In other such embodiments at least one set of the plurality of chambers may be fluidically connected in series. In this context embodiments can have sets comprising at least 2, or comprising at least 5, or comprising at least 10 chambers. Furthermore, in such embodiments, devices may comprise at least two sets of chambers, or comprise at least 4 sets of chambers, or comprise at least 5 sets of chambers. For example, there may be 8 sets of chambers, each set having 12 chambers. In some embodiments some, or all, of the sets of chambers are not mutually fluidically connected. In other embodiments some, or all, of the sets of chambers are mutually fluidically connected. This may be done using further chambers not shown in the Figures.

In preferred embodiments of a device as claimed herein the device is not a fluidic device as disclosed in any one of international patent applications PCT/EP2024/066936, PCT/EP2024/067688, and PCT/EP2024/067028. For example, a device as claimed does not comprise the device wherein the substrate is an integrally formed substrate comprising a chamber comprising the at least one recess and wherein the integrally formed substrate comprises a filter chamber housing a filter membrane that divides the filter chamber in a first sub-chamber and a second sub-chamber, wherein the filter membrane is not part of a bottom of a recess for cultivating 3D-cell cultures, such as for example spheroids or organoids, therein, wherein the filter membrane is a perforated membrane comprising a plurality of pores having a diameter of 10 µm or less, wherein the integrally formed substrate comprises a filter fluid channel connecting the first sub-chamber to the chamber the second chamber such that fluid can flow from the second chamber to the first sub-chamber via the first fluid channel. In some such embodiments, the filter chamber does not have any recess for cultivating 3D-cell cultures, such as for example spheroids or organoids. In some such embodiments the recess not comprising the filter membrane or not present in the filter chamber may be a recess as defined in the current disclosure. Additionally, or alternatively, a device as claimed herein does not include the device having

In a second aspect a method of cultivating a 3D-cell cluster in the at least one recess of a device as claimed herein is provided. The method comprises adding cells and a fluid medium to the at least one recess. The method may further comprise replacing at least part of the fluid medium with further fluid medium. Preferably the 3D cell cluster comprises a spheroid or organoid.

In a third aspect a method of subjecting a 3D-cell cluster in the at least one recess of a device as disclosed herein to a drug is provided. Preferably the 3D cell cluster comprises a spheroid or organoid.

In a fourth aspect use of a device as disclosed herein for cultivating or investigating a 3D-cell cluster, such as for example comprising spheroids or organoids, is provided.

### BRIEF DESCRIPTION OF THE DRAWINGS

For a better understanding of the claimed aspects and embodiments, and to show more clearly how they may be carried into effect, reference will now be made, by way of example only, to the accompanying schematic drawings not drawn to scale, in which:
Figs. 1A and 1B illustrate a device according to principles disclosed herein. Fig. 1A shows a perspective view of the device. Fig. 1B provides a cross-sectional frontal view of the device in Fig. 1A;
Fig. 2 shows a cross-sectional frontal view of an alternative device.
Fig. 3 shows a cross-sectional frontal view of another alternative device.
Figs. 4A and 4B illustrate devices with one or more recesses in a chamber. Fig. 4B is a cross-sectional view of Fig. 4A along the plane 413;
Figs. 5A to 5C show an embodiment of a device according to the disclosed principles. Fig. 5A shows the chamber 520 in detail. Fig. 5B shows a perspective view of the device. Fig. 5C provides a cross section of the device along a plane 513.
Figs. 6A to 6J illustrate examples of devices having multiple chambers;
Figs. 7A and 7B illustrate flow diagrams of methods of use of devices disclosed herein;

### DETAILED DESCRIPTION OF THE EMBODIMENTS

The claimed aspects and embodiments will be described with reference to the Figs.

The detailed description and specific examples, while indicating implementations, examples and embodiments of the devices, systems and methods and uses, are intended for purposes of illustration and are not intended to limit the scope of the disclosure or claims. The same or similar reference numerals are used throughout the Figs. to indicate the same or similar parts unless otherwise indicated.

The disclosure provides a device, system and methods and uses for cultivation, investigation and experimentation of a three-dimensional cell cluster, also referred to herein as a 3D-cell cluster. Such cluster may be part of or contained in a 3D-cell culture. The devices and systems may sometimes be referred to as 'culturing device or system' or 'microfluidic device or system'.

The term 'for culturing a 3D-cell cluster' is intended to encompass the growing and/or maintaining of such clusters for a defined amount of time for various kinds of purposes such as for example experimentation, biological investigation, disease investigation, disease treatment; drug efficacy testing, and others.

A 3D-cell culture, also referred to as a 3D-cell cluster, is an artificially created environment in which biological cells are permitted to grow or interact with their surroundings in all three dimensions.

One type of 3D-cell cluster and 3D-cell culture comprises spheroids. Spheroids for various experimentation typically have diameters in the range of 50 µm to 1000 µm. Often used spheroids have diameters in the range of 100 µm to 700 µm, or in the range of 100 µm to 500 µm.

Another type of 3D-cell culture comprises organoids. An organoid is a miniaturized and simplified version of an organ produced in vitro in three dimensions that mimics the key functional, structural, and biological complexity of that organ. It is derived from one or a few cells from a tissue, embryonic stem cells, or induced pluripotent stem cells, which can self-organize in three-dimensional culture owing to their self-renewal and differentiation capacities. Organoids typically have sizes with dimensions or diameters in the range of 100 µm to 5000 µm. Often cultivated organoids have dimensions in the range of 500 µm to 2000 µm.

The terms "Fluid" or "fluidic medium" are used to indicate liquids, such as for example watery liquids, and/or, sometimes, gasses such as e.g., oxygen or carbon dioxide. Such liquids can, and typically do, contain compounds dissolved or dispersed therein, such as for example nutrients for the cell-clusters or drugs to be tested on the 3D-cell clusters for effectiveness etc.

The term "recess", also referred to herein as "microwell" are herein used to refer to spaces or volumes partly or completely enclosed by a material of the device. The recesses defined herein are configured to contain a 3D-cell cluster at least partly therein.

The terms "chamber", "further chamber" and "fluid channel" are herein used to refer to spaces or volumes that are partly or completely enclosed by a material of the device. They are configured to comprise the fluids and/or transport the fluids.

The term "fluidic connection" as used herein means that for two parts connected with such fluidic connection fluid can pass from one part to the other via the connection. The two parts are then fluidically connected.

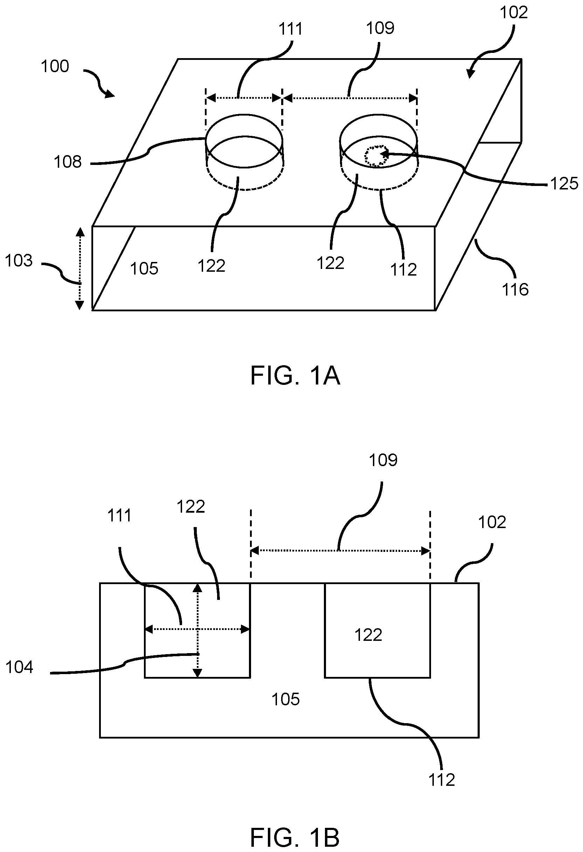

Figs. 1A and 1B illustrate a device 100 according to currently disclosed principles. The device may be referred to as a 3D-cell cluster culturing device.

The device of Figs 1A and 1B will first be used to describe several general features and characteristics of a substrate having recesses according to the disclosure. Such general features and characteristics can then be implemented in different embodiments of devices as disclosed herein.

For example, while in some embodiments, the device of Figs. 1A and 1B can represent an entire device as disclosed herein, in other embodiments there can be additional parts and features within a device as will become apparent from the disclosure hereinafter.

With reference to Figs. 1A and 1B again, the device 100 includes a substrate 105 having a thickness 103 and a substrate surface 102, which may be referred to as the top substrate surface 102 when the device is in use as for example described hereinafter. The device 100 comprises two recesses 122 representing "the at least one recess" defined in a device disclosed herein. The two recesses extend within the substrate 105. Each of the recesses has a recess depth 104 measured from the first substrate surface 102 to the recess bottom 112. Even though two recesses are shown in the device 100, a different number of recesses, such as only one, or more than two may be present in other devices according to the current disclosure. Any recess 122 has a recess opening 108 extending within the first substrate surface 102 with a recess opening circumference 107 enclosing a recess opening area 110. In this case the recess opening circumference 107 of each recess 122 is circular with a diameter 111. However, other shapes may be used.

Each recess 122 has a cylindrical shape with a cylinder axis perpendicular to the first substrate surface 102. Each recess bottom 112 has a flat surface. The two recesses 122 are spaced apart according to a pitch 109 measured along a spacing direction in the plane of the first substrate surface 102. Such pitch 109 is typically larger than the diameter of the recess opening measured along the spacing direction so that the spaced apart recesses do not merge into one. Each recess has a recess depth 104 defined as the distance from the first substrate surface to the recess bottom (preferably the largest distance). The substrate 105 has a second substrate surface 116, which is, but need not be, parallel to the first substrate surface 102. A thickness 103 of the substrate can be defined at the location of one or more of the two recesses as the distance between the first substrate surface 102 and the second substrate surface 116. The thickness typically is larger than the recess depth such that the substrate typically defines the recess bottom. A spheroid 125 is shown to be housed at least partially in one of the two recesses 122.

The example of Figs. 1A and 1B has cylindrically shaped recesses. This is preferred, but not necessary. In other embodiments recesses of many different shapes can be used so long as they are able to at least partly, and preferably substantially entirely, house the 3D-cell clusters described herein, such as e.g. spheroids and/or organoids. Some shapes are more preferred than others for various reasons. For example, recesses with symmetrical or regular shapes can often be more easily and/or more reproducibly manufactured than irregularly shaped ones. They may also be better suited for arrangement in arrays disclosed hereinafter. Furthermore, sometimes the shape of such 3D-cell clusters can be influenced (e.g. steered) by the shape and/or dimensions of a recess, i.e. for example, 3D-cell clusters such as spheroids, with substantially circular cross-sections, may be better cultivated in correspondingly or similarly shaped recesses. This may also help to increase uniformity of size among a plurality of such grown clusters by using a plurality of same size and shape recesses.

Thus, in preferred examples recess with regular shapes are used. For example, Fig 1A illustrates a cylindrically shaped recess with a uniform circular cross-section. Alternatively, or additionally any recesses of a device may have the shape of a cone or frustum of a cone, or of a prims or frustum of a prism, where a prism is a polyhedron comprising an n-sided polygon base. Preferably, the larger base of a frustrum forms the recess opening. In yet other examples the recess has a spherical shape or even different shapes. Cone shaped recesses may allow easier manufacture using casting or injection molding manufacturing techniques.

The recess opening may be related to the shape of a recess. In the example shown in Fig. 1A the recesses have a cylindrical shape with a smoothly curved side surface and a circular cross-section. However, as mentioned, recesses with circumference shapes different form circular can be used. In some devices the recess opening may have a polygonal shape, such as when the recess is of the prism or frustum of prims shape type. Exemplifying regular polygon shapes as recess opening shapes include, but are not limited to: square, pentagon, hexagon, octagon etc.

### Recess opening dimensions

The desired cross-sectional dimension 111 and cross-sectional area of a recess, measured parallel to the plane of the first surface, for example and preferably, in the plane of the first surface, can be defined by an effective diameter 111 and an effective area. Accordingly, the effective diameter and associated effective area may be defined as the diameter and area of the largest circle that can be drawn within the circumference of the relevant cross-section.

The effective diameter and area allow specification of values for recess openings and shapes with cross sections that are irregular or non-circular. If the cross section is specified within the first substrate surface, the cross section represents the recess opening.

In devices disclosed herein, each recess has an effective diameter of at least substantially 50 µm. This would amount to a recess opening effective area of at least substantially 0.002 mm². In some embodiments, wherein the recess cross section is of circular shape, such effective diameter and area represent actual dimension and area of the recess. In alternative, or additional embodiments, the effective diameter may deviate from the actual dimension and area, such as for example when recess areas are not of circular shape. A recess with the defined effective diameter typically provides a recess with a cross section large enough to at least partially house even the typical smaller-sized spheroids.

Preferably the effective area is at most substantially 20 mm². These areas typically define a recess large enough to house even the larger type organoids. In preferred embodiments the effective area is not more than substantially 25 mm², or even not more than 20 mm². This allows cultivation of 3D-cell clusters of several mm diameter in the recesses. For example, a cylindrical recess with circular recess opening of 20 mm² recess area would have a diameter of about 5 mm, which is large enough for substantially sized organoids to be contained in such recess.

### Recess depth

In devices disclosed herein each recess has a recess depth of at least substantially the value of the effective diameter. Thus, the recess depth may be at least substantially 50 µm. For example, of a 3D-cell cluster that just fits into the recess opening 108 of Fig. 1A, a substantial part may be located within the recess and below the first substrate surface 102. With such recess depth, a 3D-cell cluster with a size comparable to the recess effective diameter may still be contained within the recess for a substantial part and thereby be better retained at its location during experimentation compared to a situation in which the spheroid is not in a recess, e.g. on top of a flat surface. It will be clear that recesses with larger recess depth may be able to provide a similar advantage for larger size 3D-cell clusters. In preferred embodiments the recess depth is at least substantially the same as the effective diameter. The deeper the recesses for a same 3D cluster, the more pronounced the retaining effect can be. Thus, by design of the recess, the location of a 3D-cell cluster may be more stationary in the device. For example, movement of the cluster during manipulation of any fluidic medium surrounding a cluster may be reduced compared to when a cluster is not in a recess and situated on a flat surface.

### Recess design

Each recess may be appropriately sized and shaped according to principles as disclosed herein to house or support a particular 3D-cell culture. For example, a recess may have a cylindrical shape with a circular or semicircular (oval or regularly polyhedral) shape cross-section and a recess depth as defined herein. In other words, the shape and size of a recess may be chosen based on the intended 3D-cell cluster to be cultivated in the recess. Exemplifying embodiments may be defined as follows. For spheroids of typical sizes in ranges given herein before, the diameter may be in the range of 50 µm to 2000 µm, or in the range of 100 µm to 1000 µm, or in the range of 100 µm to 500 µm, or 50 µm to 500 µm. A preferred range is 100 to 1000 Some of these embodiments may also be suitable for cultivating organoids. Organoids typically are larger than spheroids. Accordingly, embodiments of devices for organoid experimentation may have recesses with diameters in the range of 50 µm to 5000 µm, or in the range of 500 µm to 3000 µm, or in the range of 500 µm to 2000 µm.

As a working example, each recess may have a recess depth in the range of 50 µm to 1000 µm, preferably in the range of 50 µm to 500 µm and a recess effective diameter in the range of 0.5 µm to 1000 µm. For example, a recess may have a recess depth of 500 µm and a recess effective diameter of 700 µm. A recess with a recess effective diameter, in the range of 50 µm to 200 µm may be most desirable for cultivating spheroids. A recess with a recess effective diameter in the range of 200 µm to 1000 µm may be most desirable for cultivating organoids.

Matching dimensions of a recess to the typical dimensions of a 3D-cell cluster localizes them in the substrate and first substrate surface of the device. Furthermore, during manipulation of fluid in the recesses the 3D-cell clusters are less likely to be removed from their (initial) location. One or more of these advantages may in turn may make experimentation (exposure to drugs or other compounds or analysis) with the 3D-cell cluster easier as the location of the recess in the device may be known. Alternatively, or additionally, in some instances the size and shape of a 3D-cell cluster during cultivation may be controlled (e.g. limited) to some extent by the choice of size and shape of the recess. This may enable experimentation in which reproducibility or statistical analysis is important.

### Recess bottom

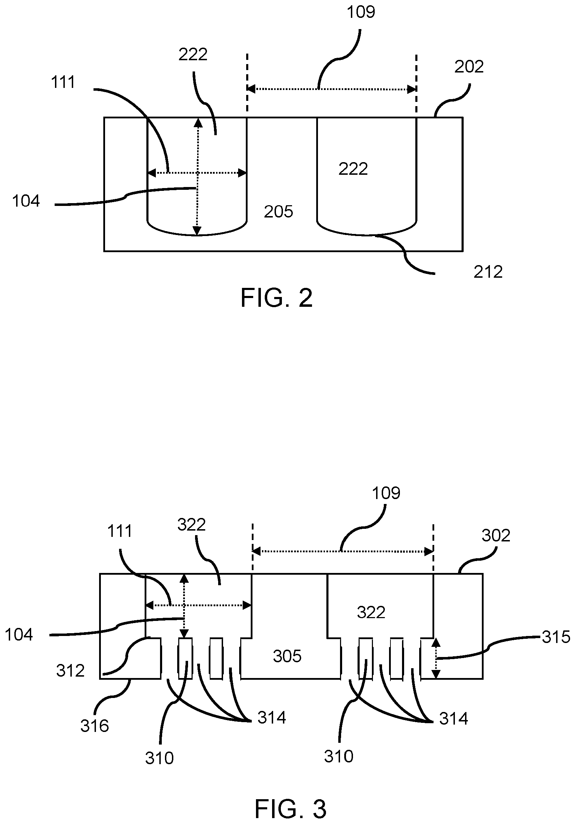

The device 100 of Figs 1A and 1B has recesses 122 with flat bottoms 112. This is not necessary, and differently shaped bottom surfaces can be used. Preferred shapes are according to continuous surfaces such as for example concave as for example indicated by the concave shape of recess bottom 212 shown in the front view of Fig. 2 of an alternative device having again two recesses 222 in a substrate 205 with top substrate surface 202 that is otherwise similar to that of the device of Fig. 1A.

In some embodiments at least one, or a plurality, of recesses comprises a permeable bottom permeable for fluid or a fluidic medium. An exemplifying device is one like that of Fig. 1A, but in which the recess bottoms 112 are perforated with at least one, but preferably a plurality of pores (not shown in the Fig. 1A. A front view cross-section of a device 300 is shown in Fig. 3. The device 300 is like that of Fig. 1A, but with some differences explained with reference to Fig. 3. The recess bottom 312 in each of the recesses 322 has a plurality of pores 314 (of which for clarity reasons only 3 are shown per recess) extending through the substrate bottom portion 310 from the recess bottom 312 to the second substrate surface 316. Per recess there may be 50 or more pores, or 100 or more pores, or 200 or more pores, or even 500 or more pores. A pore has a pore length equal to the recess bottom thickness 315 of a bottom portion of the substrate 305. A pore 314 typically has a pore diameter smaller than the recess diameter. The recess bottoms 312 are flat but other shapes, such as e.g. the concave one of recesses 222 can be used.

The pore diameter may be tuned to types of cells of a 3D-cell cluster to be prevented from passing through a pore. By way of example, if the desired cell (or cell of interest) is a leukocyte (white blood cell), then the pore diameter may be at most 3 µm, e.g., at most 2 µm or less as these cells can pass through extremely small pores. As another example, cancer cells (of potential interest) are larger than this, and the pore diameter may be set accordingly (e.g. as indicated herein before), to facilitate retention of such cells by the perforated membrane. Hao, Si-Jie, et al. "Size-based separation methods of circulating tumor cells." Advanced drug delivery reviews 125 (2018): 3-20 provides examples of cancer cell sizes that can be used to determine or set a maximum pore diameter, as would be readily understood by the skilled person.

Each pore can have a pore diameter sufficiently large to allow passage of fluid containing constituents such as small molecules, drugs and proteins. The theoretical minimum diameter of each pore may therefore be dependent upon fluidic medium and its contents. For example, for water-based fluids, to ensure sufficient/fast flow of fluid, each pore may have a diameter of at least substantially 1 µm or 2 µm . In addition, the pore diameter may be at most any one of the following values 20 µm, 10 µm, 9 µm, 8 µm, 6 µm, and 5 µm. As a working example, each pore may have a pore diameter in the range of 5 µm to 10 µm or of 5 µm to 10 µm. The pore diameters of different pores need not be identical for all pores.

A permeable bottom portion 310, such as e.g. a perforated recess bottom 310 , may be advantageous for one or more reasons. For example, a flow of fluid can be established through such pores for cultivation purposes, such as e.g. for refreshing nutrients and/or removing metabolic waste, or for exposure of the cell cluster to compounds such as drugs to be tested etc. Furthermore, the flow may be used to keep a 3D-cell cluster more secure in a recess during any manipulation of fluidic medium. The pores may be used to provide a recess with testing compounds.

In preferred embodiments the substrate bottom portion, which is a part of the substrate comprising the recess bottom, comprises a membrane. Such membrane can be particularly advantageous in combination with the pores as described herein. Thus, for example and with reference to Fig. 3, the substrate portion 310 of each of the recesses 322 and comprising the pores 314 can have a thickness 315 such that it provides a membrane 310. Any membrane as defined herein may be configured as follows. A preferred thickness is in the range of 5 to 100 µm, but other thickness can be used. More preferably the thickness is in the range of 5 to 40 µm, or in the range of 10 to 40 µm. Most preferably the thickness is in the range of 10 to 20 µm. For thicker membranes pores may be more difficult to make e.g. using laser perforation and thinner membranes may be weaker and more difficult to make using injection molding, 3D printing or casting.

### Plurality of recesses

In preferred embodiments, the at least one recess of a device comprises a plurality of recesses mutually spaced apart across the surface. The number of recesses in a device may be any suitable number and can vary depending upon the use-case scenario. For example, there may be 5 or more, 10 or more, 20 or more, 50 or more 100 or more, 200 or more recesses.

Each recess may be configured or usable for culturing a separate 3D-cell cluster. The plurality of recesses may be arranged in a random or irregular pattern, but, preferably, they are arranged in a linear array or two-dimensional array along rows and columns defined in the plane of the first substrate surface. The rows and columns may extend in mutually perpendicular directions, but this is not necessary, and alternatively they can extend in directions at oblique angels. The recesses may be spaced apart according to pitches along row and column directions. These pitches may be the same or different.

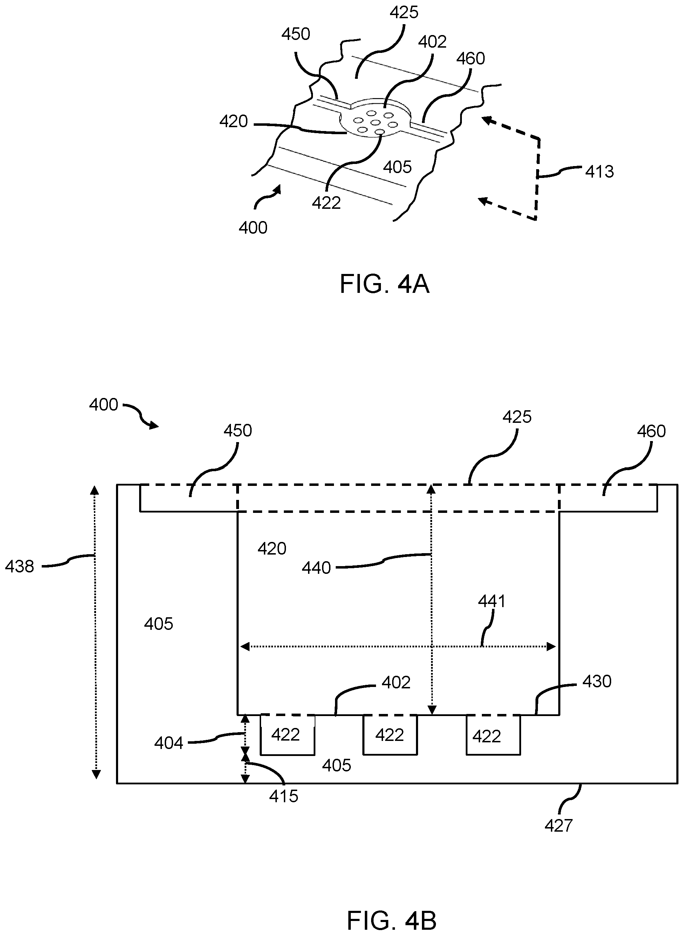

The device of Fig. 1A shows a one-dimensional array of 2 recesses. Fig. 4 shows part of a substrate 405 of a device 400 wherein the substrate 405 has a two-dimensional array of 7 recesses 422 in a first substrate surface 402. The recesses are arranged along rows and columns in the plane of the first surface 402 along mutually non-perpendicular directions at angles of substantially 60 degrees. The pitches 409 (not shown) are the same for rows and columns. While the substrate portion 405 shows the presence of two fluidic channels 450 and 460 in fluidic communication with the recesses, these may be omitted in other embodiments.

Since the locations of each recess 420 in the array and therewith the device may be known and can be tracked using analysis equipment, experimentation with a plurality of 3D-cell clusters may be easier as described herein before.

The shapes and/or sizes of a plurality of recesses may be, but need not be, the same. Each of the shapes and sizes may be designed according to principles as described herein. In some embodiments, the shape and size of each recess of a plurality of recesses, or of a subset of the plurality of recesses, are substantially the same. The plurality or subset of the plurality of recesses may then be used to perform statistical experiments on a plurality of 3D-cell clusters subjected to substantially the same cultivation and experimentation conditions. Alternatively, such size and shape of each of a plurality of recesses, or of a subset of the plurality of recesses differ such that one type of experiment performed on different sizes 3D-cell cultures in the plurality of recesses may be performed with one device.

### Chamber design

In preferred embodiments the device comprises at least one chamber for holding a fluidic medium. A chamber typically is larger than a recess and is designed to have a bottom comprising the first substrate surface having the at least one recess as defined herein. It is contemplated that a portion of a fluid channel, where such portion has the at least one a recess as defined herein, is to be designated a chamber as defined herein and this is regardless of any design of such fluid channel. Thus, for example, a fluid channel having a length with a constant cross-sectional shape and area along the length and having the at last one recess in a portion along the length, is considered to comprise a chamber as defined herein. Figs. 4A and 4B are used to illustrate some features and characteristics of a chamber that can be implemented in devices as disclosed herein. Thus, a chamber 420 may be defined within the substrate 405 extending over a chamber depth 440 from a further substrate surface 425 to the chamber bottom 430. The chamber shape may be chosen as desired and for example may have similar shapes as described for the recesses. Preferably, and as shown in Figs 4A and 4B the chamber shape is substantially cylindrical with a cylinder axis perpendicular to the further surface 425. The chamber depth 440 may be in the range of 0.5 mm to 3 cm. For a smaller volume chamber its depth may be in the range of 0.5 to 2 mm. For a larger volume chamber its depth may be in the range of 0.5 to 3 cm. The chamber has an effective diameter 441 that can be defined as the effective diameter for the recess. Accordingly, the chamber effective diameter is the diameter of the largest circle that can be drawn within the circumference of a chamber cross-section taken perpendicularly to the direction in which the chamber extends from the further substrate surface.

The chamber bottom 430 comprising the first substrate surface 402 with 7 recesses 422 arranged in the 2D-array described herein above. Although the embodiment has 7 recesses in the chamber 422, other embodiments can have a different number of recesses such as one, or a plurality as described herein before. Although the embodiment shows a distribution of recesses according to a particular two-dimensional array any other distribution also can be chosen. The number, size and pitch of the recesses may be chosen based on the chamber effective diameter 441 so long as the chamber bottom area is large enough to encompass all the recesses defined in the first substrate surface 402.

The fluid channels 450 and 460 may be advantageous if the device 400 is a closed device in which the chamber is closed off from the top. For example, the device 400 may comprise a cover plate (not shown) over the surface 425. The cover plate may be clamped, glued or otherwise adhered to the first substrate surface to provide a leak tight closure of the fluid channels and, optionally, the chamber 420. The fluid channels may be fluidically connected to a fluid inlet and fluid outlet of the device. An alternative device does not have any such fluid channel or any fluid channels. For example, the device can be an open device, or one of which a cover plate can be removed. In such case fluid manipulation can be done via the chamber opening 408 in the further surface 425.

Since the recesses are relatively small, fluidic medium manipulation during experimentation for providing/removing/exchanging such medium in an individual recess may be difficult. It may then be advantageous to have the recess as part of a larger volume chamber in fluidic connection with the recess via the recess opening. The larger size of the chamber and larger fluid media volumes may allow easier manipulation of the fluidic medium therein, and therewith in the recesses of the chamber. Furthermore, if there are a plurality of recesses in a chamber, each 3D-cell cluster within a recess can be provided with similar fluidic medium conditions upon simple manipulation of the medium in the chamber. The latter may be advantageous for statistical analysis.

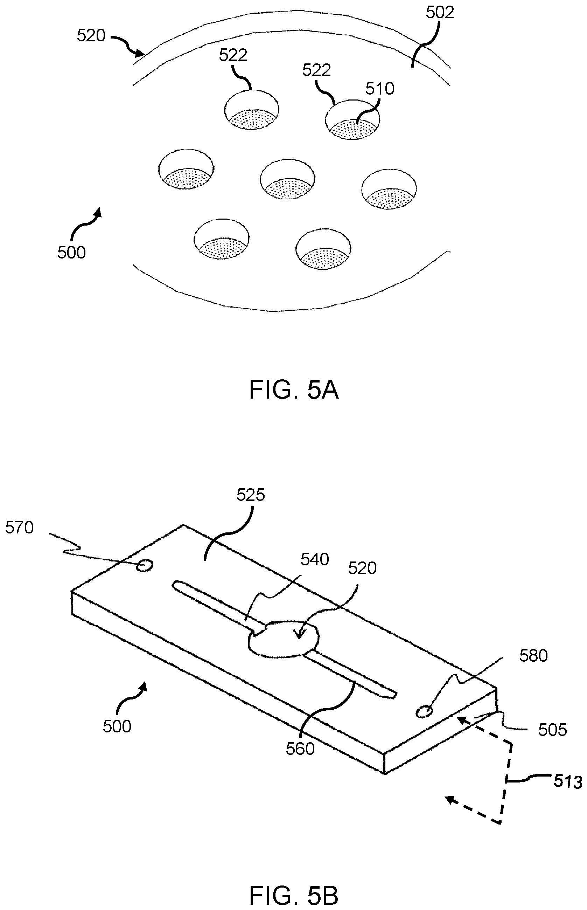

In some embodiments each recess, or a subset of recesses, of the plurality of recesses within a chamber, has a bottom permeable for fluid. For example, such recess bottom may be a perforated bottom, either in membrane form or not, as described herein before. For example, and with reference to Fig. 5A, the chamber 520 comprises the surface 502 having therein 7 recesses 522 each of which has a perforated membrane 510.

To illustrate how recesses with a permeable bottom can be implemented in a device as disclosed herein Figs. 5A to 5C are used. In the device 500 the chamber 520 is part of the substrate 505 with the first substrate surface 502 and the recesses 522 therein. Each of the recesses 522 has a perforated membrane 510 at its recess bottom. The substrate 505 comprises fluid channels 540 and 560 extending parallel to and at the side of the first further surface 525 of the substrate 505 and fluidically connected to the chamber 520. The substrate 505 and perforated membrane are designed such that there is a further chamber 520B that can contain fluid medium and that is fluidically connected to the chamber 520 via the pores of the perforated membranes 510. The substrate 505 comprises fluid channels 570 and 580 fluidically connected to the further chamber 520B. Parts of the fluid channels 570 and 580 extend parallel to the second further surface 527 at the bottom side of the 505 and further parts of the fluid channels extend through the substrate 505 from the second further surface side 527 to the first further surface side 525 ending in openings the first further surface 525. The openings may serve as fluid inlet and/or fluid outlet. A first cover plate 542 is pressed against or adhered to the first further surface 525 such that the fluid channels 540 and 560 are closed off fluid leak free. The plate has openings 543 and 545 that fluidically connect to the channels 540, 560 and has openings 547 and 549 that fluidically connect to the openings of fluid channels 570 and 580. A second cover plate 544 is pressed against or adhered to the second further surface 527 and closes the fluid channels 570 and 580 in a fluid leak free manner.

The openings in the first cover plate 542 can be used to access the fluid channels, i.e. to provide or withdraw fluid medium from the channels and therewith the chambers connected to the channels. For example, the openings and fluid channels may be connected to a pump or pump system to cause flow of fluid to and from the recesses via the chamber 520 and further chamber 520B. Fluid channels 540 and 560 may be used to cause a flow across the chamber 520. Fluid channels 570 and 580 may be used to cause fluid flow across further chamber 520B. Using one or more fluid channels 540 and 560 and one or more fluid channels 570 and 580 a fluid flow through the recesses and through the perforated membranes 510 may be established. A flow from the chamber to the further chamber may help keep 3D-cell clusters located in recesses 522.

In an alternative design (not shown), the substrate 505 and perforated membranes 510 are designed such that the second substrate surface 516 is flush with upper walls of the fluid channels 570 and 580. This may provide a smaller volume further chamber 520B.

The device 500 including the first and second cover plates 542 and 544 can be a closed device. All of the chambers and recesses are then only accessible via the respective openings and fluid channels. Alternatively, the device is an open or partially pen device. Accordingly, the first cover plate 542 may be removable to provide direct access to a chamber or recess or the first cover plate is omitted entirely, or there is a whole in the plate at the location of the chamber 520. In such cases also the fluid channels 540 and 560 may be omitted as direct access to the chamber 520 and its recesses is already available. The direct access allows manual or automated fluid medium manipulation using pipetting.

In some embodiments the total pore cross-sectional area of a plurality of pores in any bottoms of a recess or a plurality of recesses may be chosen to allow a fluid flow (e.g. in volume per time) that is sufficient to cause fluidic medium provision/removal/or exchange for cultivation or test experimentation through the pores. In some examples, this total pore area is equal to or larger than the cross-sectional area of any fluid channel used to provide or withdraw fluid from a chamber or recess or a plurality of recesses. Hence, a generated pressure to cause a fluid flow may be kept low even when some of the pores become obstructed for fluid flow for any reason. A reduced pressure may help prevent rupture of a relatively thin perforated membrane as described herein below if the pores are within such a membrane.

Any substrate disclosed herein, including those of embodiments described with reference to Figs 4A, 4B and 5A to 5C can have a substrate thickness measured from the first further surface to the second further surface. The thickness may be at least substantially 10 µm. Preferred thicknesses are in the range of 10 µm to 5000 µm, preferably in the range of 10 µm to 2500 µm. It will be clear that the thickness will be related to the design of any chambers, recesses, membranes and fluid channels. For example, with reference to Fig. 4B, the chamber depth 440, recess depth 404 substrate thickness 438 and the thickness 415 of the recess bottom may be interrelated. These measures may be used to design a device having a particular chamber, recess and recess bottom as defined herein.

### Plurality of chambers

In some embodiments a device as disclosed herein has a plurality of chambers each chamber then comprising at least one, but preferably a plurality, of the recesses as disclosed herein.

A device with a plurality of chambers can be implemented in many ways, some of which will now be described in more detail.

One implementation provides for embodiments in the form of a multi well plate, of which one, a plurality, or all, of the wells are chambers as defined herein with one, a plurality, or all of the wells comprising the at least one recess as defined herein. Such multi well plates may be advantageous for high throughput screening as many of the 3D-cell clusters may be cultivated in one single device while it may be easier to locate and/or retain them at the locations of the recesses each one resides in. If there is a plurality of recesses in a chamber, the plurality of recesses may again for example be used for the statistical analyses as described herein.

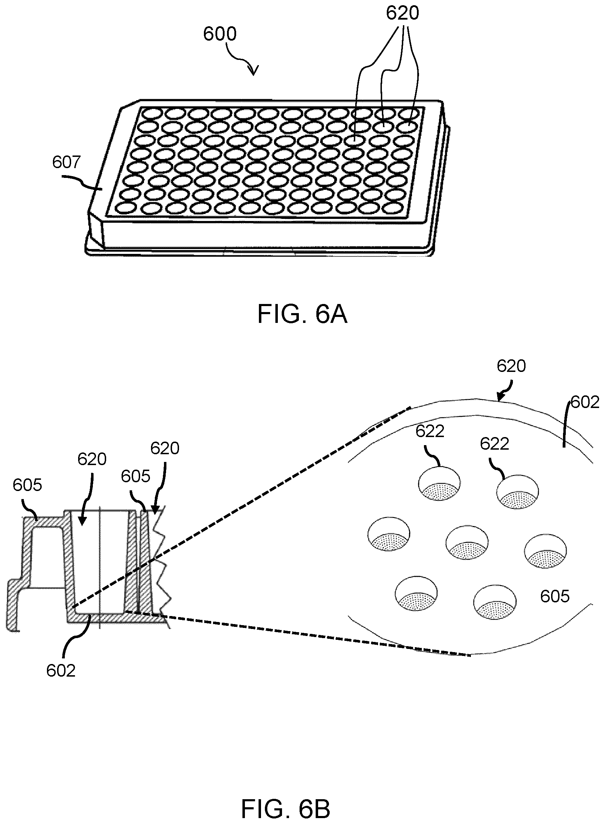

The devices described with reference to Figs. 6A to 6D are used as first embodiments to show how recesses as disclosed herein can be implemented in standard multi well plate format. Such standard well plate format is generally used in the field of biological and medical experimentation using standard experimentation and analysis equipment known in the art.

The illustrated devices have a well plate, for example according to ASME Y14.5N-1994, having a body 607 comprising 96 wells 620 therein. Other standards may be used. Body 607 may for example be made of polystyrene or polypropylene, but other materials can be used. The well plate typically is around 9 cm wide and 13 cm long and between 1.5 cm and 4 cm high depending on whether it has so called normal or deep wells. The wells are distributed across the body surface 625 of the body 607 with a same pitch 609 in both the column and row directions of an array of wells. However, pitches 609 may be mutually different. The wells 620 typically have circular cross section and their diameter typically is dependent on the available body dimensions, pitch and number of wells in a row or column. The dimensions, such as diameter and depth, of wells in such plates is dependent on the dimensions of the well plate, pitches of wells in rows and columns as well as the number of wells per row and/or column. For example, typical dimensions of wells in such standard format plates include a well depth in the range 0.5 to 5 cm; and a well diameter of 0.5 to 5 cm. As a descriptive and working example only, the plates of Figs 6A to 6D have 96 wells with a pitch of 9 mm in row and column directions. The well diameter is 4 mm and the well depth is 1.3 mm. Other dimensions may be used.

Fig. 6B shows a vertical cross-sectional view of an edge section of a first embodiments using the standard multi well plate. A substrate 605 is in this case an integral part of or the same as the body 607. The bottom of one, a plurality, or each well 620 has a bottom surface 602 and comprises 7 recesses 622 distributed across the first substrate surface 602. Preferably, all of the wells have the recesses. The recesses 622 may be defined as described herein before, such as for example they may be those defined as described with reference to Figs. 4A and 5A. Since the wells 620 have only well openings at their top, the recesses have no perforated bottom. The number, recess opening diameter and recess pitches may be chosen according to desire as long as the chosen design fits the well bottom area given by the 4 mm diameter wells. In this example the recesses are provided in the same material as that of the body 607 since the substrate 605 and body 607 are an integrated part. Hence, simple manufacturing using for example injection moulding may be used. Preferably such material is a transparent material such that optical analysis from top side or, more preferably, bottom side is enabled for example with a system as described herein below.

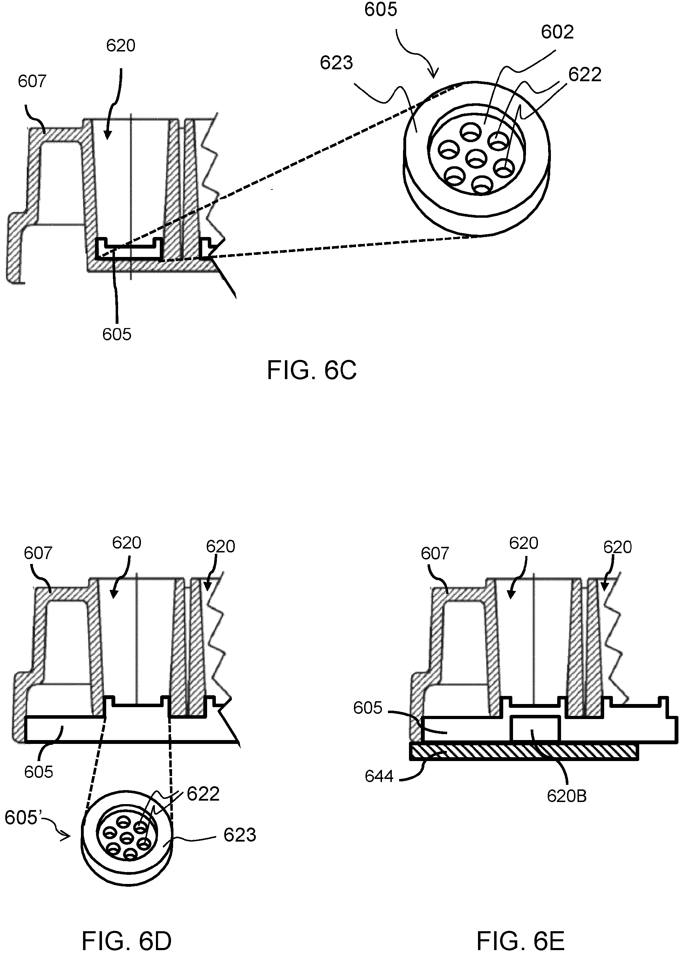

Fig. 6C shows a vertical cross-sectional view of and edge section of s second embodiment using the standard multi well plate. In this embodiment the body 607 is separate from any substrate 605. In one, a plurality, or each well there is a substrate 605 having a first substrate surface 602 that forms the bottom of the well. The first substrate surface 602 comprises 7 recesses 622. The substrate 605 shows a rim 623 extending from the first substrate surface 602, but this is not essential, and the rim may be omitted. Any substrate 605 may be an insert to the wells of body 607 and may be permanently fixed at the bottom of a well or may be removable from the well. This design may be advantageous as the substrate material having the wells may be made of a material different from the material of the body 607. Thus, for example while the body 607 may still be made using the standard materials such as polystyrene or polypropylene giving stiffness and support to the device, the substrate material can be made of a material more suitable for 3D-cell cultivation. Preferred substrate materials in such design are elastomeric materials, such as e.g. polysiloxane based elastomeric materials as described herein. The number and precise design of the recesses per well may be chosen according to principles described for the embodiment of Fig. 1A. The substrate inserts may be added during manufacturing or during use. This design setup may be advantageous in terms of flexible well configuration using standard well plates. Thus, for example, wells may be equipped with differently designed recess configurations, i.e. some with larger recess dimensions and others with smaller recess dimensions for growing different types of 3D-cell clusters. Not all wells need to be provided with the substrate inserts, etc.

Fig. 6D shows a vertical cross-sectional view of and edge section of a third embodiment using the standard multi well plate format, but with wells without a bottom defined by the body 607. Instead, the bottoms are formed by the plurality of first substrate surfaces 602 of the substrate 605. The bottom of one, a plurality or all, of the wells are formed by a first substrate surface 602 of protrusions 605' of substrate portion 605. Each first substrate surface 602 comprises 7 recesses 622 as described herein, such as for example that of Figs 4A. Other recess configurations can be used. The substrate protrusion 605' shows a rim 623 extending from the surface 602 of protrusion 605', but this is not essential, and the rim may be omitted. The protrusion shape and diameter and those of the well bottom opening are designed such that when the protrusion is inserted in the well opening as shown, a leak tight seal is obtained so that the well can contain a fluid without leakage. In contrast to, for example the implementation of Fig. 6C, a plurality, or all, of the well bottoms are part of one integrated substrate 605. This design may reduce the burden of assembly of a plurality of separate substrates 605 into separate wells. Instead, the body 607 and substrate 605 may be assembled in a single step.

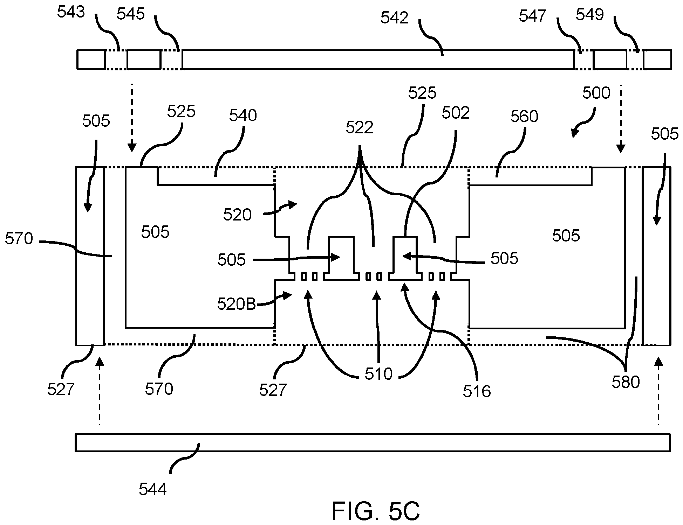

Fig. 6E shows a fourth embodiment that is similar to that of Fig. 6D, with the difference that in this case substrate 605 comprises a further chamber 620B underneath one, a plurality, or all of the well bottoms. And, although not shown for clarity, each of the recesses in the well bottom is permeable, preferably perforated, as defined herein such that the chamber 620 is fluidically connected to the further chamber 620B for purposes as defined herein. For example, the perforated bottom may be implemented as described with reference to Fig. 5A to 5C. Thus, in a preferred variant, the substrate 605 has a perforated membrane 610 as defined herein such as for example described for the embodiment described with reference to Fig. 5A to 5C. The further chamber 620B may be fluidically connected to fluidic channels 670 and 680 that are also located and defined within the substrate 605. For a plurality of such further chambers 620B in a substrate where each one is associated with a well 620, several or all of these further chambers 620B may be fluidically connected to each other in series or in parallel fashion for example via any fluidic channels. Such interconnection may be realized along a row direction, a column direction or both directions of the array of wells 620 in the plate. In the embodiment shown in Fig. 6E, interconnection is in the direction perpendicular to the plane of drawing. Any further chambers 620B and fluidic channels in the substrate 605 are sealed leak tight by a bottom plate 644 which can be clamped or adhered to (e.g. using glue or other adhesive forces) to the substate 605 as for example also described for the embodiment of Fig. 5C.

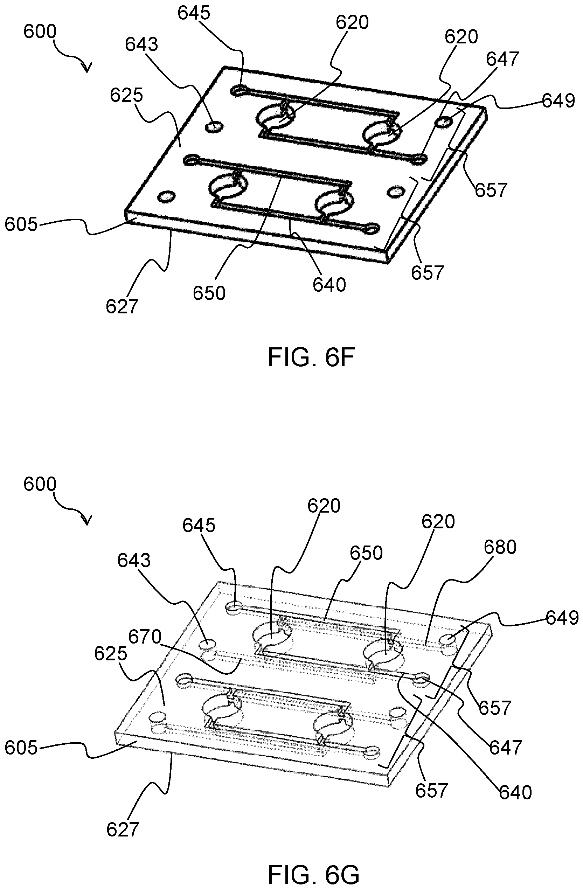

To illustrate how a device with multiple chambers where each chamber has the at least one recess as defined herein can be implemented not using a standard well plate, Figs. 6F to 6J are used. The device 600 of Figs. 6F and 6G comprises a substrate 605 having two identically designed pairs 685 of each two chambers 620 fluidically connected in parallel using fluidic channels 640, 650, 670 and 680. For each chamber 620, the substrate 605 comprises a perforated membrane 610 as described for the chamber 520 of Fig. 5C. Such membranes 610 have not been shown in the Figs. 6F and 6G for clarity. In some embodiments the membranes are an integral part of the substrate, in other embodiments the membranes may be provided as separate parts inserted in the chambers. In any case, the fluid channels 640 and 650 are directly fluidically connected to the chambers 620 and the fluid chambers 670 and 680 are directly fluidically connected to the further chambers 620B. The fluidic channels 640 and 650 are fluidically connected to openings 645 and 647 and the fluidic channels 670 and 680 are fluidically connected to the openings 643 and 649. Hence, per pair two chambers 620 can be accessed in parallel using the relevant channels and openings as for example already indicated for one channel described with reference to Figs. 5A to 5C. Any first cover plate 642 and a second cover plate 644 to be adhered to the first further surface 625 and the second further surface 627 of the substrate have not been drawn for clarity but can be part of the device. For example, a cover plate 644 can be used against the surface 627 to close the bottom channels and further chamber 620B. The first cover plate may also be used against surface 625 but may be dispensed with if needed as described for the cover plate of the embodiment of Fig. 5A to 5C.

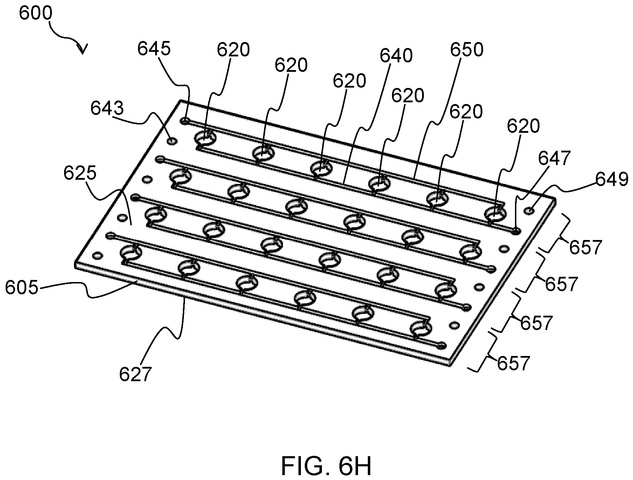

The design of the embodiment of the device of Fig. 6F and 6G can be extended to one that includes more than two chambers per parallel fluidic connection. In such case the substrate format and dimensions may be the same as that of the standard well plate such that although the device is not based on a standard well plate, it still can be used in existing analysis and experimentation equipment used for the standard well plates. One example is shown in Fig. 6H where there are 4 sets 657 of each 6 parallel fluidically connected chambers 620 using fluidic channels and openings as defined for the pairs 658 in Figs. 6F to 6G. Thus, a 24-chamber plate may be implemented. However, plates with any other number of chambers may be implemented as well.

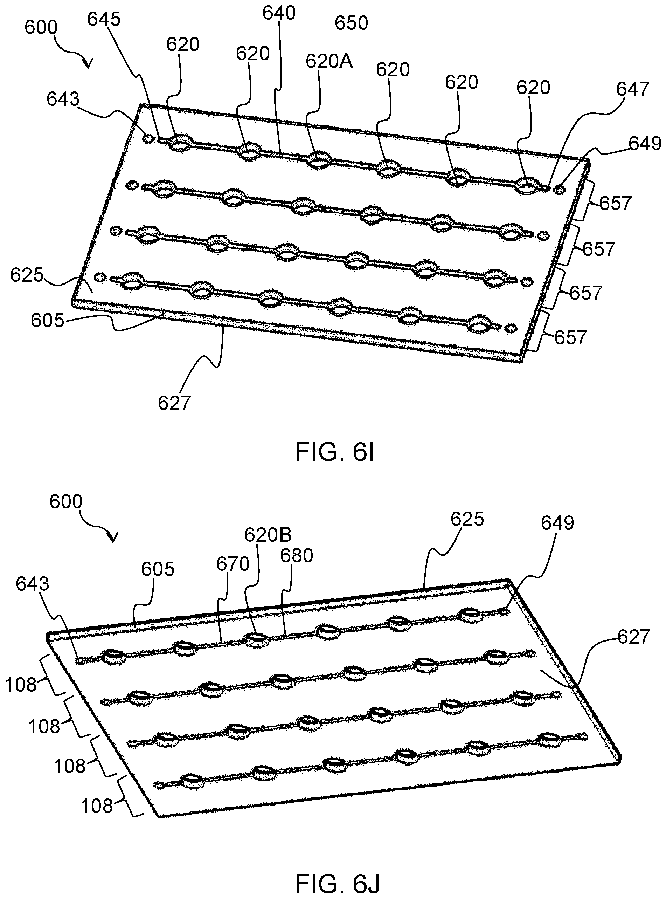

Multi chamber plates with fluidically interconnected chambers may be implemented using parallel connection of chambers as described herein above but may also be implemented with series connection. Thu, Figs. 6I and 6J show a top and bottom view of a device 600 with 4 sets of each 6 chambers interconnected in series using channels 640, 650, 670 and 690. It is noted that in this case a channel 640 for one chamber (e.g. 620A) is the same channel 650 for a chamber directly fluidically connected to that chamber (e.g. 620A). In other words, whereas in parallel fluidic connection of chambers in devices 600 of Figs. 6F to 6H, a channel 640 of a set 657 may be used to supply each chamber of the set with fluid while a channel 650 of the set 657 may be used to drain fluid from those chambers, in the series fluidic connection of chambers of device 600 of Figs. 6I and 6J, the channels 640 and 650 perform both these functions at the same time but for different chambers of a set. In other words, depending on which chamber is considered, a channel 640 may be a supply channel or a drainage channel at the same time. Similar considerations hold for the channels 650, 670 and 680 as will be apparent to those skilled in the art. As for the embodiments shown in Figs. 6F to 6H, the channels of device 600 shown in Figs. 6I and 6J can be accessed via openings 643, 645, 647 and 649 as described herein.

In preferred embodiments of devices as disclosed herein having fluidically connected chambers in a set of chambers, such as for example the devices 600 in Figs. 6F to 6J, any set of chambers comprises at least 2 chambers, or comprises at least 5 chambers, or comprises at least 10 chambers. In some embodiments the device comprises only one set of chambers. In other embodiments the device comprises at least two sets of chambers, or comprises at least 4 sets of chambers, or comprises at least 5 sets of chambers. For example, there may be 8 sets of chambers, each set having 12 chambers. In some embodiments series fluidic connection is preferred over parallel fluidic connection. Series fluidic connection may be more space efficient and therefore allow more chambers per surface area of the substrate or device. In some embodiments some, or all, of the sets of chambers are not mutually fluidically connected. In other embodiments some, or all, of the sets of chambers are mutually fluidically connected. This may be done using further chambers not shown in the Figures.

Further embodiments in which the recesses and chambers with recesses may be implemented in substrates and devices are provided in the patent applications PCT/EP2024/067688, PCT/EP2024/066936 and PCT/EP2024/067028 which are all incorporated by reference herein in their entirety. These documents provide further designs for combining multiple chambers that are fluidically connected via parallel or series layout. Some of these documents describe how fluid barriers can be implemented to control flow in such multi chamber fluidic connections. Such flow barriers can also be used in devices of the current disclosure.

In preferred embodiments of a device, substrate or body, according to the current disclosure, such device, substrate or body does not have the at last one recess in a configuration such that the at least one recess is in fluidic contact of a perforated membrane that is itself not part of a recess. a plurality of recesses where one of the plurality of recesses is fluidically connected to another of the plurality of recesses via a fluidic channel. For example, in such case the one of the plurality of recesses can be part of a chamber, having at last one chamber comprising the at least one recess

### Body and substrate

In some embodiments of a device as described herein comprises an integrally formed body formed by the substrate, also sometimes referred to herein as "monolithic substrate" or monolithic body, where "integrally formed" means formed of one piece of material. For example, the embodiments of Figs 4, 5, 6B, and 6F to 6J may have such monolithic substrates. For example, the integrated body (thus including the substrate) can be made of a relatively stiff material (e.g. thermoplastic plastic) or the integrated body can be made of a relatively less stiff material (e.g. elastomeric material). A device with an integrally formed body may benefit from a simpler manufacturing process as the integrally formed body can be made in one manufacturing step implementing many features at once.

In other embodiments, the device as described herein comprises a body and substrate as assembled, but separate, parts. In such cases, the body and substrate can be made of separate pieces of material. For example, the embodiments of Figs. 6C to 6E may have a body and substrate of separate pieces of material. In preferred embodiments the body is made of a relatively stiff material (e.g. thermoplastic plastic) while the substrate can be made of relatively less stiff material (e.g. elastomeric material). In some preferred embodiments, the relatively stiff body comprises any chambers whereas the substrate comprises the first substrate surface including any recess and perforated membrane if any is present. A device with assembled body and substrate allows for more design freedom for a device in terms of material and mechanical properties.

### Fluid channel

A fluid channel as defined herein may also be referred to as a fluid duct. They are volumes at least partly enclosed by the device materials capable of holding or transporting fluid or fluid medium. The transporting of fluid may include supplying fluid to one or more a recesses and/or chambers and/or draining fluid from one or more recesses and/or chambers. The fluid channels in exemplary embodiments described herein are typically defined in the substrate and/or body of a device. However, this is not necessary. In some embodiments fluid channels are entirely, or partially, defined or comprised within any cover plates or other parts of a device.

### Materials

Devices as disclosed herein may be manufactured using materials with properties as disclosed hereinafter. While these materials are preferred materials, other materials can be used.

Preferably, the materials used are biocompatible in the sense that they have a low toxicity to cells and any 3D-cell clusters and cultures. For example, substrate materials are preferably biocompatible as the substrate comprises the recesses in which the 3D cell clusters will reside. Additionally, or alternatively, materials are at least to some extent, but preferably, substantially transparent for visible and/or UV light. For example, some or all parts of a device, such as e.g. the substrate and/or bottom thereof, a body, a chamber bottom, or a recess bottom, or a top and/or cover plate are made of a transparent material. This may enable the use of the device in regular optical analysis equipment as in such equipment oftentimes optical inspection occurs from the bottom side of a device when in use. Hence, a substrate or at least its bottom, and, if any, a bottom cover plate, are preferably made of such transparent material.

A substrate of a device, whether as an integrally formed body or separate from a body, can comprise or consist of, or can be, partly or entirely, made of, an organic polymeric material. The organic polymeric material may for example comprise one or more polymers chosen from the group consisting of thermoset polymers, thermoplastic polymers, elastomeric polymers, or combinations thereof. Such polymers may be co-polymers of different monomers or not. They may be blockcopolymers or not.

In some embodiments the organic polymeric material is a non-elastomeric material. For example, the non-elastomeric polymeric material comprises or, alternatively, consists of one or more polymers chosen from the group consisting of: non-elastomeric thermoset polymers and non-elastomeric thermoplastic polymers. Such polymers include, but are not limited to: cyclic olefin polymer (COP) or a cyclic olefin copolymer (COC), polycarbonate or polystyrene, polycarbonate, polyether ether ketone (PEEK), acrylonitrile butadiene styrene (ABS), polyetherimide (PEI), and polyethersulfone (PES). These non-elastomeric organic polymers may provide the organic polymeric material with a relatively high stiffness at temperatures of normal use conditions of the device (e.g. temperatures of 40 degrees Celsius or less).

The non-elastomeric organic polymers may be advantageously used in or for parts of a device that are at least partly intended to provide shape integrity to the device. For example, these materials may be used as part of a body of a device, whether that body is an integrally formed one or a body separate from the substrate of a device. Devices, whether having an integrally formed body or a body separate from substrate typically comprise, and that make use of standard well plates, typically comprise such materials. In some embodiments of devices having separate body and substrate, the substrate is formed from a more biocompatible material than the body. This may help cells or 3D-cell clusters to preferentially adhere to the substrate. For example, silicone, e.g., silicone modified in the manner described hereinafter, may form the substrate whereas a polymeric material for which the biological material has less affinity, such as a polymeric material comprising one or more materials chosen from the group consisting of polycarbonate, polyether ether ketone (PEEK), acrylonitrile butadiene styrene (ABS), polyetherimide (PEI), and polyethersulfone (PES), may form the body. For instance, the less affinity material may form the sidewall(s) of any chambers of a body while the substrate forms the bottom of the chambers.

In some embodiments the organic polymeric material is an elastomeric material. For example, the elastomeric polymeric material comprises or, alternatively, consists of one or more polymers chosen from the group consisting of: elastomeric polymers; elastomeric thermoset polymers and elastomeric thermoplastic polymers. Suitable elastomeric polymeric materials for substrates and, if any, membranes and perforated membranes, are based on elastomers such as: polysiloxane (such as for example polyalkylsiloxane), polybutadiene rubber (PBR), styrene-ethylene-butadiene-styrene elastomer (SEBS), thermoplastic polyurethane (TPU), and thermoplastic silicone vulcanizate (TPSiV). Others may be used too. Preferred elastomeric materials include polysiloxane-based elastomers which is sometimes referred to as "silicone" based elastomer. Such elastomers include polyalkylsiloxane based polymers such as for example polydimethylsiloxane (PDMS) based polymers with or without modifications to improve cell cultivating properties. In terms of such properties, in some embodiments desired silicone-based elastomers are described in for example WO2019015988A1 and WO2021/058657 which are incorporated by reference herein in their entirety. The modified silicone elastomers, such as for example the carboxylic acid modified elastomers, disclosed therein may be conveniently used in an injection molding process for preparing any substrate or integrally formed body. Elastomeric materials for the substrate are described, for example, in WO2021058657, WO2019015988. These materials are also suitable to provide good adhesion of upper and lower cover plates made of for example glass or polar plastics such as poycarbonate.

Silicone and polybutadiene are readily functionalized, e.g. via cross-linking with fatty acids, to render them biocompatible, e.g. via application of a protein such as fibronectin to the fatty acid-functionalized surface of the elastomeric material. This, together with their elastic properties, makes such elastomeric materials particularly appropriate for inclusion in the substrate.

The elastomeric material may, for example, be a fatty acid-crosslinked elastomeric material, for example a fatty acid-crosslinked silicone and/or a fatty acid-crosslinked polybutadiene. Such a fatty acid-crosslinked elastomeric material can be particularly suitable for the substrate and or recess and/or recess bottom, owing to the polarity introduced by the carboxylic acid groups of the fatty acid.

The fatty acid-crosslinked elastomeric material can be manufactured in any suitable manner, such as by bulk-modifying the elastomeric material with the fatty acid. It is noted that bulk-modified elastomeric materials suitable for inclusion in the substrate and or recess and/or recess bottom are described, for example, in WO2021058657 and WO2019015988.

In some embodiments, at least some of the carboxylic acid groups of the fatty acids crosslinked to the elastomeric material, e.g. to the silicone and/or polybutadiene, are arranged at surface(s) of the fatty acid-crosslinked elastomeric material.

In such embodiments, the fatty acids included in the fatty acid-crosslinked elastomeric material may render the surface(s) of the elastomeric material particularly compatible with polar species, such as cell culture protein(s), e.g. fibronectin, and/or a polar environment to which the biological material is exposed during the testing.

In some embodiments the recess bottom comprises a cell culture protein for contacting the biological material. The cell culture protein, e.g. fibronectin, may be arranged, for instance, on surface(s) of the fatty acid-crosslinked elastomeric material at which the carboxylic acid groups are provided.

The following exemplary commercially available elastomeric/soft materials, which can be transparent or translucent if no dies are added, and are food contact approved, can also be used: Medalist MD-53253 (TPE Teknor Apex); Medalist MD-53273 (TPE Teknor Apex); Mediprene 500602 M-03 (TPE Hexpol); Texin Rx T85A (TPU Covestro); BioSpan^{®} (F SPU | PUR); BioSpan^{®} (SPU | PUR); BJB Polyurethane F-116 A/B | TSU; BJB Polyurethane F-126 A/B | TSU; BJB Polyurethane F-131 A/B | TSU; BJB Polyurethane M-3115 REV 1 A/B | TSU; BJB Polyurethane M-3125 A/B | TSU; CELLENE MC2248 | TPE; CELLENE MC2265 | TPE; CELLENE MC3038 | TPE; CELLENE MC3050 | TPE; CELLENE MC3061 | TPE; CELLENE MC3226 | TPE; CELLENE MC3239 | TPE; CELLENE MC3261 | TPE; Dynaflex^{™} G2706-1000-00 | TPE; Dynaflex^{™} G2711-1000-00 | TPE; Elastocon^{®} 2860L | TPE; Filter-bond^{™} E-3264 | TS; FLEXCHEM^{™} 3551-02 | PVC, Flexible; FLEXCHEM^{™} 4051-02 | PVC, Flexible; FLEXCHEM^{™} 4551-02 | PVC, Flexible; FLEXCHEM^{™} 5051-02 | PVC, Flexible; FLEXCHEM^{™} 5551-02 | PVC, Flexible; FLEXCHEM^{™} 6051-02 | PVC, Flexible; FLEXCHEM^{™} 6551-02 | PVC, Flexible; Medalist^{®} MD-12130 | TPE; Medalist^{®} MD-12130H | TPE; Medalist^{®} MD-12140 | TPE; Medalist^{®} MD-12140H | TPE; Medalist^{®} MD-12150 | TPE; Medalist^{®} MD-12150H | TPE; Medalist^{®} MD-12f150S | TPE; Medalist^{®} MD-12160 | TPE; Medalist^{®} MD-12160H | TPE; Medalist^{®} MD-12170 | TPE; Medalist^{®} MD-12170H | TPE; Medalist^{®} MD-12243 | TPE; Medalist^{®} MD-12337 | TPE; Medalist^{®} MD-12340 NAT | TPE; Medalist^{®} MD-12342 | TPE; Medalist^{®} MD-12344 | TPE; Medalist^{®} MD-12350 | TPE; Medalist^{®} MD-12352 | TPE; Medalist^{®} MD-12362 | TPE; Medalist^{®} MD-125 | TPE; Medalist^{®} MD-130 | TPE; Medalist^{®} MD-13240 | TPE; Medalist^{®} MD-135 | TPE; Medalist^{®} MD-145 | TPE; Medalist^{®} MD-155 | TPE; Medalist^{®} MD-17365 | TPE; Medalist^{®} MD-225 | TPV; Medalist^{®} MD-32045 | TPE; Medalist^{®} MD-32245 | TPE; Medalist^{®} MD-36048 | TPE; Medalist^{®} MD-37063 NAT | TPE; Medalist^{®} MD-42245 XRD1 | TPE; Medalist^{®} MD-42245 XRD3 | TPE; Medalist^{®} MD-74357 XRD1 | TPE; Medalist^{®} MD-74357 XRD2 | TPE; Mediprene^{®} 500120M | TPE; Mediprene^{®} 500200M | TPE; Mediprene^{®} 500250M | TPE; Mediprene^{®} 500300M | TPE; Mediprene^{®} 500350M | TPE; Mediprene^{®} 500400M | TPE; Mediprene^{®} 500434M | TPE; Mediprene^{®} 500450M | TPE; Mediprene^{®} 500484M | TPE; Mediprene^{®} 500520M | TPE; Mediprene^{®} 500534M | TPE; Mediprene^{®} 500584M | TPE; Mediprene^{®} 500600M | TPE; Mediprene^{®} 500634M | TPE; Mediprene^{®} 500650M | TPE; Mediprene^{®} 500684M | TPE; Mediprene^{®} 500700M | TPE; Monprene^{®} RG-10160H | TPE; ProvaMed^{®} TPE 1120 | TPE; ProvaMed^{®} TPE 1160 | TPE; RABALON^{®} PJ4300C | TPE; RABALON^{®} PJ5300C | TPE; RABALON^{®} PJ6300C | TPE; RABALON^{®} PJ7300C | TPE; SkinFlex 15 F-115 A/B | TSU; SkinFlex BR-60; BRUSHABLE A/B | TSU; T-Blend^{®} TPE-F22 | SEBS; THERMOLAST^{®} M TM3LFT (Series: MC/LF) | TPE; THERMOLAST^{®} M TM3MED (Series: MC/tl) | TPE; THERMOLAST^{®} M TM3RST (Series: MC/RS) | TPE; THERMOLAST^{®} M TM4LFT (Series: MC/LF) | TPE; THERMOLAST^{®} M TM4MED (Series: MC/tl) | TPE; THERMOLAST^{®} M TM4RST (Series: MC/RS) | TPE; THERMOLAST^{®} M TM5LFT (Series: MC/LF) | TPE; THERMOLAST^{®} M TM5MED (Series: MC/tl) | TPE; THERMOLAST^{®} M TM6LFT (Series: MC/LF) | TPE; THERMOLAST^{®} M TM6MED (Series: MC/tl) | TPE; THERMOLAST^{®} M TM7LFT (Series: MC/LF) | TPE HERMOLAST^{®} M TM7MED (Series: MC/tl) | TPE; UNISOFT SPECIAL^{™} DS-35A-CL-M-01 | SEBS; UNISOFT SPECIAL^{™} DS-55A-CL-M-01 | SEBS; Versaflex^{™} G2705 N | TPE; Versaflex^{™} HC 1100-40 Translucent EU | TPE; Versaflex^{™} HC 1348 Natural | TPE; Versaflex^{™} HC MT317 | TPE; Versaflex^{™} HC MT555 | TPE; Versaflex^{™} OM 1040X-1 | TPE; CELLENE MC2248 | TPE; CELLENE MC2265 | TPE; CELLENE MC3038 | TPE; CELLENE MC3050 | TPE; CELLENE MC3061 | TPE; CELLENE MC3226 | TPE; CELLENE MC3239 | TPE; CELLENE MC3261 | TPE; ChronoPrene^{™} 25A | TPE; ChronoPrene^{™} 40A | TPE (CardioTech International, Inc.); Dryflex^{®} 500300S | TPE; Dryflex^{®} 500350S | TPE; Dryflex^{®} 500400S | TPE; Dryflex^{®} 500450S | TPE; Dryflex^{®} 500500S | TPE; Dryflex^{®} 500550S | TPE; Dryflex^{®} 500600S | TPE; Dryflex^{®} 500650S | TPE; Dryflex^{®} 500700S | TPE; Dynaflex^{™} G2701-1000-02 | TPE; Dynaflex^{™} G2706-1000-00 | TPE; Dynaflex^{™} G2709-1000-00 | TPE; Dynaflex^{™} G2711-1000-00 | TPE; Dynaflex^{™} G2712-1000-02 | TPE; Dynaflex^{™} G2730 | TPE; Dynaflex^{™} G2755-1000-00 | TPE; Dynaflex^{™} G2755C | TPE; Dynaflex^{™} G6713-0001 | TPE; Dynaflex^{™} G6713C | TPE; Dynalloy^{™} GP 7810-60T | TPE; Dynalloy^{™} GP 7810-70T | TPE; Dynalloy^{™} OBC8200-BT50 | TPE; Estane^{®} 58123 TPU | TPU-Polyether; Evoprene^{™} 019 | SBS; Evoprene^{™} G 925 | SEBS; Evoprene^{™} G 936 | SEBS; Evoprene^{™} G 942 | SEBS; Evoprene^{™} G 958 | SEBS; Evoprene^{™} G 966 | SEBS; Evoprene^{™} G 967 | SEBS; Evoprene^{™} G 968 | SEBS; Evoprene^{™} G 969 | SEBS; Evoprene^{™} G 970 | SEBS; Evoprene^{™} GC 5685 | SEBS; Evoprene^{™} GC 5686 | SEBS; Evoprene^{™} GC 5687 | SEBS; Evoprene^{™} GC 5688 | SEBS; Evoprene^{™} GC 5689 | SEBS; Evoprene^{™} GC 5690 | SEBS; GLS 422-126 | TPE; GLS 458-140 | TPE; GLS 458-141 | TPE; GLS 458-142 | TPE; K-Prene HYFLEX HF 15 | MPR; K-Prene HYFLEX HF 20 | MPR; K-Prene HYFLEX HF 25 | MPR; K-Prene HYFLEX HF 30 | MPR; Medalist^{®} RG-38052 XRD1 | TPE; megol^{®} PUG 10 | SEBS; megol^{®} PUG 60 | SEBS; megol^{®} TA 60 | SEBS; Monprene^{®} RG-10130 | TPE; Monprene^{®} RG-10140 | TPE; Monprene^{®} RG-10150 | TPE; Monprene^{®} RG-10160 | TPE; Monprene^{®} RG-10170 | TPE; Monprene^{®} RG-15130 | TPE; Monprene^{®} RG-15140 | TPE; Monprene^{®} RG-15150 | TPE; Monprene^{®} RG-15160 | TPE; Monprene^{®} RG-15170 | TPE; Monprene^{®} RG-18240 | TPE; Monprene^{®} RG-18250 | TPE; Monprene^{®} RG-18260 | TPE; Monprene^{®} RG-18270 | TPE; Monprene^{®} RG-19221 NAT | TPE; Monprene^{®} RG-19255 | TPE; Monprene^{®} RG-20140 | TPE; Monprene^{®} RG-20160 | TPE; Monprene^{®} RG-20170 | TPE; Monprene^{®} RG-29068 NAT | TPE; Monprene^{®} RG-29240 XRD1 | TPE; RABALON^{®} MJ4300C | TPE; RABALON^{®} MJ5302C | TPE; RABALON^{®} MJ6301C | TPE; RABALON^{®} MJ7301C | TPE; RAYPRENE^{®} NB221-S4050 | TPE; RAYPRENE^{®} NB221-S4051 | TPE; RAYPRENE^{®} NB221-S4052 | TPE; RAYPRENE^{®} NB221-S4053 | TPE; tefabloc^{®} TO 132 | TPE; Telcar^{®} TL-83-F943D22-NT BLU | TPE; THERMOLAST^{®} K TF2CGT (Series: FC) | TPE; THERMOLAST^{®} K TF3BTL (Series: FC/AP) | TPE; THERMOLAST^{®} K TF3CGT (Series: FC) | TPE; THERMOLAST^{®} K TF3STE (Series: FC/CS) | TPE; THERMOLAST^{®} K TF4AAB (Series: FC/HE/tl) | TPE; THERMOLAST^{®} K TF4BTL (Series: FC/AP) | TPE; THERMOLAST^{®} K TF4CGT (Series: FC) | TPE; THERMOLAST^{®} K TF4STE (Series: FC/CS) | TPE; THERMOLAST^{®} K TF5AAC (Series: FC/HE/tl) | TPE; THERMOLAST^{®} K TF5BTL (Series: FC/AP) | TPE; THERMOLAST^{®} K TF5CGT (Series: FC) | TPE; THERMOLAST^{®} K TF5STE (Series: FC/CS) | TPE; THERMOLAST^{®} K TF5WHA (Series: DW/H) | TPE; THERMOLAST^{®} K TF6AAF (Series: FC/HE/tl) | TPE; THERMOLAST^{®} K TF6BTL (Series: FC/AP) | TPE; THERMOLAST^{®} K TF6CGT (Series: FC) | TPE; THERMOLAST^{®} K TF6STE (Series: FC/CS) | TPE; THERMOLAST^{®} K TF6WCS (Series: DW/CS) | TPE; THERMOLAST^{®} K TF6WHA (Series: DW/H) | TPE; THERMOLAST^{®} K TF6WHB (Series: DW/H) | TPE; THERMOLAST^{®} K TF7AAC (Series: FC/HE/tl) | TPE; THERMOLAST^{®} K TF7BTL (Series: FC/AP) | TPE; THERMOLAST^{®} K TF7CGT (Series: FC) | TPE; THERMOLAST^{®} K TF7WHB (Series: DW/H) | TPE; Topolymer^{®} 8201-B | TPE; Versaflex^{™} FFC 2882-50 EU | TPE; Versaflex^{™} FFC 2882-50 | TPE; Versaflex^{™} G2708 N | TPE; Versaflex^{™} GP 2810-20N | TPE; Versaflex^{™} GP 2810-30N | TPE; Versaflex^{™} GP 2810-40N | TPE; Versaflex^{™} GP 2810-50N | TPE; Versaflex^{™} GP 2810-60N | TPE; Versaflex^{™} GP 2810-70N | TPE; Cawiton^{®} MT920 | SEBS; Cawiton^{®} MT930 | SEBS; Cawiton^{®} MT940 | SEBS; Cawiton^{®} MT950 | SEBS; Cawiton^{®} MT960 | SEBS; Cawiton^{®} MT970 | SEBS.

The parts of a device as disclosed herein may be made of a material that is of one type of polymer or of a blend or composition of polymers as described herein. Materials and combinations of materials, including some falling within the categories described herein above, for use in devices have been disclosed in international patent applications PCT/EP2024/067688, PCT/EP2024/066936 and PCT/EP2024/067028 all of which are incorporated by reference herein in their entirety.

One advantage of having a substrate made of an elastomeric organic material, whether as integrally formed body or separate from the body of a device, resides in that cell cultivation may benefit.

Another advantage resides in that glass or plastic cover plates can be adhered to the substrates almost or substantially leak free and without complicated adhesion materials such as glue. This may also be useful to implement releasable top cover plates of devices.

Some of the elastomeric organic materials or polymers are thermoplastic (e.g. thermoplastic polymer elastomer (TPE)) and some are not. The TPE's can be relatively easily used in injection molding or 3D-printing manufacturing processes. This can benefit the manufacturing of a device in terms of complexity, cost and/or reproducibility. The polysiloxane-based polymers often are not of the TPE type. Nevertheless, while some of them are better suited for use in a casting process for making a substrate, others are better suited for use with an injection molding process for making the substrate.

Some of the elastomeric organic materials and polymers allow laser ablation for shaping. For example, a membrane of some of these materials, such as of the silicone-based polymers, may be perforated using laser ablation methods described in for example WO2023/046744 which is incorporated by reference herein in its entirety.

Some of the elastomeric organic materials and polymers are optically transparent in the way defined herein above. This is beneficial for devices designed for use with optical inspection equipment, such as visible light microscopes with or without fluorescent staining. To assess the outcomes of any staining experiments, polysiloxane-based elastomeric organic polymers are again particularly advantageous as they show little autofluorescence. Thus, in some preferred examples, the substrate and/or other features of the device may be formed from a polysiloxane based elastomeric organic material or polymer such as those described herein before.

Typically, the elastomeric organic polymers provide a lower stiffness to parts made of them than do the non-elastomeric organic polymers described hereinbefore. Nevertheless, some of the elastomeric materials and polymers have relatively higher stiffness and some of them have a relatively lower stiffness, where the relatively higher stiffness is high enough to provide a shape integrity to the parts made of them at normal device use temperatures as indicated herein before. Accordingly, a device having separate elastomeric substrate and body, of which the substrate has relatively lower stiffness, which typically is the case when using silicone-based elastomer material for the substrate, typically has a body of relatively higher stiffness. The materials of the parts of a device may thus be chosen according to these stiffness principles making use of e.g. stiffness measurement methods or stiffness data as known in the art. For example, in some embodiments the body of a device may be made of the non-elastomeric organic material as described herein before. In other embodiments the body may be made of an elastomeric material with stiffness high enough to provide the shape integrity. Examples include StyreneButheneEthyleneStyrene (SEBS) copolymers, optionally mixed polyethynene (PE). These may have shore A (hardness) of 28 and (moduluss E = 3,0 tot 3,5 MPa). In yet further embodiments, a bottom cover plate is preferably part of the device to provide the shape integrity by being made of the materials providing the relatively higher stiffness.

The stiffness principles may also be used in choice of materials for devices having integrally formed body. In such case either the body has the relatively higher stiffness, or, for example, one or more suitably stiff cover plates are used.

### Cover plates

Cover plates may be made of inorganic polymeric materials such as glass and/or of organic polymeric materials (plastic) as e.g. further described herein below. Suitable plastics include, but are not limited to, the non-elastomeric thermoplastic or thermoset materials as for example described for body materials herein below. For example polystyrene, polypropylene, polycarbonate etc. Preferably they are transparent for visible and/or UV light to allow optical investigation of the contents of recesses and/or chambers. Preferably, cover plates are made of a relatively stiff material for ease of handling if they are removable or for providing improved integrity to a device if a substrate is relatively less stiff such as for example maybe the case for substrates made of elastomeric materials.

The cover plates described herein may be formed of any suitably transparent material, such as glass, PVC or a transparent polymer, such as a cyclic olefin polymer (COP) or a cyclic olefin copolymer (COC), polycarbonate or polystyrene. In case the substrate is made of elastomeric material, one or more of the cover plates is preferably stiffer than the substrate to provide increased structural integrity.

The combination of cover plates as described with elastomeric material substrates is advantageous as it allows good mutual contact (often by sticking together) increasing leak free closure of chambers and channels and therewith fluidic devices. The sticking forces are most often low enough to be able to releasable attach or adhere the cover plates to the substrates involved. Elastomeric materials for the substrate are described, for example, in WO2021058657, WO2019015988. These materials are also suitable to provide good adhesion of upper and lower cover plates made of for example glass or polar plastics such as poycarbonate.

Silicone and polybutadiene are readily functionalized, e.g. via cross-linking with fatty acids, to render them biocompatible, e.g. via application of a protein such as fibronectin to the fatty acid-functionalized surface of the elastomeric material. This, together with their elastic properties, makes such elastomeric materials particularly appropriate for inclusion in the substrate.

The elastomeric material may, for example, be a fatty acid-crosslinked elastomeric material, for example a fatty acid-crosslinked silicone and/or a fatty acid-crosslinked polybutadiene. Such a fatty acid-crosslinked elastomeric material can be particularly suitable for the substrate and or recess and/or recess bottom, owing to the polarity introduced by the carboxylic acid groups of the fatty acid.

The fatty acid-crosslinked elastomeric material can be manufactured in any suitable manner, such as by bulk-modifying the elastomeric material with the fatty acid. It is noted that bulk-modified elastomeric materials suitable for inclusion in the substrate and or recess and/or recess bottom are described, for example, in WO2021058657 and WO2019015988.

In some embodiments, at least some of the carboxylic acid groups of the fatty acids crosslinked to the elastomeric material, e.g. to the silicone and/or polybutadiene, are arranged at surface(s) of the fatty acid-crosslinked elastomeric material.

In such embodiments, the fatty acids included in the fatty acid-crosslinked elastomeric material may render the surface(s) of the elastomeric material particularly compatible with polar species, such as cell culture protein(s), e.g. fibronectin, and/or a polar environment to which the biological material is exposed during the testing.

In some embodiments the recess bottom comprises a cell culture protein for contacting the biological material. The cell culture protein, e.g. fibronectin, may be arranged, for instance, on surface(s) of the fatty acid-crosslinked elastomeric material at which the carboxylic acid groups are provided.

### Manufacturing

The devices as described herein are relatively easily and/or reliably manufacturable using a method with a limited number of well controllable steps.

A preferred method comprises at least one step for forming the substrate disclosed herein using casting, injection molding, or 3D-printing. Preferably the injection molding method is used as that may provide for a good throughput and reliability. In some embodiments injection molding is used for the manufacture of the substrate and body, or for an integrally formed body. In other embodiments injection molding is used only for manufacturing of a substrate while a separate body is made using another method such as for example 3D printing. A casting or injection molding method may be preferred when a substrate of some of the described elastomeric materials is desired. For example, if the substrate or integrally formed body comprises a polysilicon-based elastomeric material, preferably casting and more preferably injection molding is used.

For devices having a body separate from the substrate, an assembly step may be used to combine the substrate with a body. The body can be a standard off-the-shelf body such as that of any one of the standard well plate types described herein. Alternatively, the body is made as part of the manufacturing method also using any one of a casting, injection molding, or 3D-printing method. In preferred ones of these latter variants such a body is then made using injection molding or 3D printing.

In some methods for manufacturing a device with an integrally formed body or substrate, no assembly step to combine the substrate with the body is needed. In such method, the complete integrally formed body or substrate including any recesses, and optionally including any chambers and/or fluid channels, are made of one material.

For devices having a one or more cover plates, an assembly step may be used to combine the substrate with the one or more cover plates. The substrate may be separate from the body or part of an integrally formed body.

In one preferred embodiment the manufacturing of a substrate, whether for an integrally formed body or for combination with a separate body, is done in one single method of casting, injection molding or 3D printing. Preferably the injection molding is used. In this single method all features such as any recesses, chambers if any, and fluid channels if any, are defined in the substrate after the single method. When casting or injection molding is used a suitably designed mold may be used to this end. Designing and preparation of molds for the casting or injection molding methods is known in the art.

In another embodiment the manufacturing of a substrate, as described herein before, whether for an integrally formed body or for combination with a separate body, is done by etching or otherwise removing material from a precursor substrate to thereby create one or more of the recesses, chambers if any, or fluid channels, if any. Material removal may for example be done by etching such as laser etching from a slab of material.

In the methods for manufacturing of an integrally formed body the integrally formed body or substrate may be made with casting or injection molding of a thermoplastic material, a thermset material or elastomeric material. In one variation a typical material may comprise polystyrene or polycarbonate, but others as described herein or known in the art may be used. In a preferred method casting or injection molding of an elastomeric material is used. A preferred elastomeric material may comprise any one of the silicone-based elastomer materials.

In the methods for manufacturing of a separate body and substrate, the body may be made using casting or, as preferred, injection molding of a thermoplastic or thermoset material or using 3D printing of a suitable material.

If membranes, perforated or not, are part of a substrate, preferably injection molding or casting is used for manufacture of the substrate.

In some methods the body and substrate are both prepared with injection molding as separate parts of a device. In some embodiments they are thereafter assembled to be combined in a device. In other embodiments, the injection molding process is a so called 2K injection molding process in which when one of the substrate and body has been molded in a mold system, a portion of the mold system is removed while leaving the molded substrate or body within another portion of the mold system to thereafter combine the another portion of the mold system with yet a further portion of the mold system and molding the other part of the substrate and body against the substrate or body that had been left in the mold system.

Methods of manufacture, including the 2K molding method have been described in more detail in international patent applications PCT/EP2024/067688, PCT/EP2024/066936 and PCT/EP2024/067028 all of which are incorporated by reference herein in their entirety.

### Membrane perforation

A permeable recess bottom part of any recess, especially when in membrane form, may be manufactured by providing a plurality of pores in a recess bottom. Various approaches for manufacturing a perforated (e.g., porous) recess bottom, especially in membrane form, and in particular, elastomeric perforated membrane form, are disclosed in the US Patent Application having publication number US2022/228108A1 or WO2023/046744 which are incorporated by reference herein in their entirety. The latter document also describes methods for forming pores using laser drilling in elastomeric perforated membranes. Preferably the methods are used to prepare pores in elastomeric perforated membranes improved cell culturing such as polysiloxane elastomers. Any described methods for manufacturing the perforated membrane can be incorporated into a proposed method for manufacturing the devices disclosed herein.

### Method of use

Disclosed is inter alia a method comprising cultivating a 3D-cell cluster in the at least one recess of a device as defined herein by adding cells and a fluid medium to the at least one recess. Cultivating includes but is not limited to growing the culture from cells and maintaining a grown cell culture to keep it alive for a defined period of time. To this end the recesses can be provided with cells and fluidic media needed for cultivation of the 3D-cell clusters. Such media can have nutrients and other components needed for the cultivation task at hand. Disclosed is thus use of a device as disclosed herein for cultivation of a 3D-cell cluster in the at least one recess of the device.

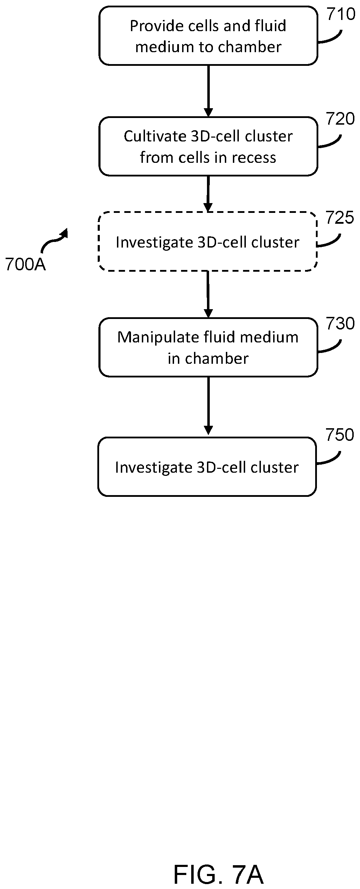

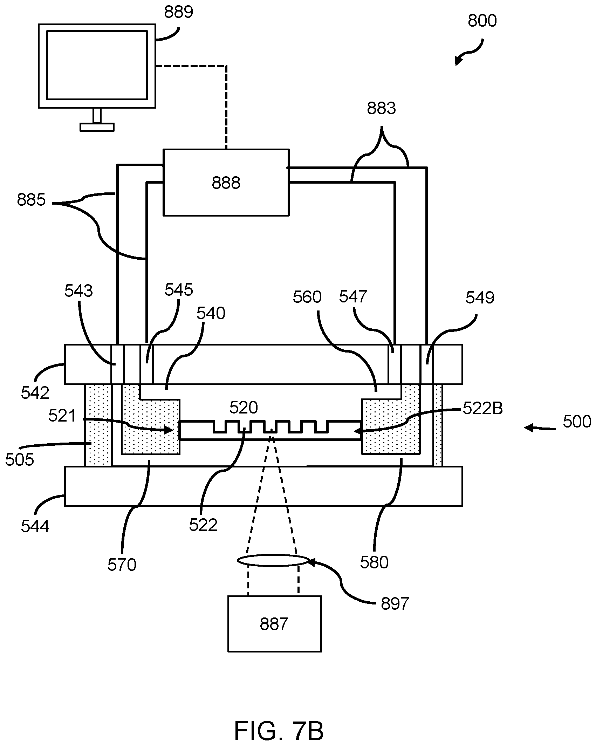

Fig. 7A illustrates such a use of devices described herein in an exemplifying cultivation method 700A. Any device as described herein may be used in the method. However, the method will be explained with reference to a device having a chamber with a plurality of recesses at its bottom arranged in an array of which recesses can be labeled according to their position in the array, such as for example the device of Fig. 4A and B or that of Fig. 5A to C. The device has a silicone elastomer substrate as defined herein and in this case is a PDMS silicone elastomer which has a largely hydrophobic surface.

The method starts with step 710 in which cells and fluidic medium are provided to the chamber. The cells are in this case suitable for growth into spheroids within the medium which in turn comprises all nutrients and components for allowing the cells to form the spheroids.

The method then continues with step 720 in which the filled chambers are exposed to conditions for suitably cultivating the cells such that they grow into spheroids. In some cases, a suitable temperature regime is applied.

In an investigation step 725 following step 720, the state of cultivated 3D-cell clusters is investigated, for example using an optical investigation system as described with reference to Fig. 8. In some cases, such investigation may involve a spheroid size determination. Other investigations may be performed alternatively or additionally. This step preferably involves labeling the cultivated 3D cell clusters according to their position on the first surface of the device, for example using the position of the recesses of an array of recesses in the chamber, and annotating the analysis results with the labels.

In a step 730 performed after step 725, a replenishment or change of fluidic medium of the chamber via manual or automatic manipulation for various purposes is performed. For example, renewed nourishment can be provided, or a drug to be tested can be provided, or a staining to investigate the state of the spheroid can be provided. In this context manual or automatic manipulation may be done via pipetting. In such case, a chamber with a chamber opening accessible by such pipetting means can be used. Alternatively, if the device has flow channels it can be used with a flow control system as described with reference to Fig. 8 to do the manipulation and the open chamber may not be needed. Providing continuous fluid medium for cultivation can be done via the top flow channels fluidically connecting to the chamber, such as fluid channels 550 and 560 of an exemplifying device of Fig 5A and 5B. Providing the drug may be done in the same way, or in the form of a therapeutic fluid such as a chemotherapy fluid that is or carries the drug for exposure to the 3D-cell cluster. For example, such drug can be provided directly to the chamber or can be introduced via an inlet and input fluid channel (where present). In the latter case such a drug can be provided via the further chamber and its fluidically connected channels and openings as for example in devices as described with reference to Fig. 5A and 5B.

While the manipulation of the medium may bring about dislocation of any 3D-cell cluster, their confinement to a recess may help prevent or reduce such dislocation such that in a follow up investigation step a same spheroid may be easily located and investigated.

In a step 740 a reinvestigation of the 3D-cell clusters is performed after the manipulation step 730. The same investigations may be performed as done in step 725, but others can be done too. In any event, in this step preferably again the results of such investigations are labeled based on the positions of the 3D cell clusters on the first surface as indicated for step 725. Based on the labels the investigation results of 3D cell clusters can be compared based on their labels. Alternatively, a label may be sued to lookup a particular 3D-cell cluster or the recess it resides in and then perform a dedicated investigation.

Fig. 8 conceptually depicts a testing system 800 according to an embodiment and is used to illustrate how devices as disclosed herein can be used for cultivation and/or analysis.

The cell culture testing system comprises one or more of a processing system 889, and a flow control system 888.

The culture device 500 of Fig. 5A and 5B is inserted in the testing system. The device assembly 500 is illustrated in cross-sectional view of Fig. 5B. The fluidic device 500 is commonly carried in a device holder which is not shown for clarity. Any holder may be used allowing and providing the function needed as for the system and as described herein. For example, the holder may comprise a clamping mechanism capable of releasably clamping the substrate 505 between the cover plates 542 and 544 such that the fluidic device is leak free. However, while the system is shown with the device 500, other devices may be used with a system 800 and such other devices may be with or without cover plates and so the clamping system is an optional one.

The device openings 543, 545, are fluidically connected to the fluid tubes 885 which in turn are fluidically connected to the flow control system. The openings 547 and 549 are fluidically connected to the fluid tubes 883 which in turn are fluidically connected to the flow control system 888.

The flow control system 888 is configured to control the flow of liquid or fluid through the fluid channels 550, 560, 570 and 580 of the fluidic device 500. In particular, the flow control system may comprise one or more pumps for controlling the flow of media or fluid through each fluid channel of the fluidic device. Thus, the flow control system can control the rate of fluid flow and/or amount of fluid through the fluidic device 500 via the input and output fluid channel(s) as required by the cases of the fluidic devices. Thus, a flow control system 888 may comprise one or more pumps for controlling the flow of liquid at least into the fluidic device. Examples are disclosed, for instance, by: Laser, Daniel J., and Juan G. Santiago. "A review of micropumps." Journal of micromechanics and microengineering 14.6 (2004): R35; Foret, Franta, and Jörg P. Kutter. "Pumps for microfluidic cell culture." (2014); or Narayanamurthy, Vigneswaran, et al. "Advances in passively driven microfluidics and lab-on-chip devices: A comprehensive literature review and patent analysis." RSC advances 10.20 (2020): 11652-11680.

The processing system 889 is configured to control the operation of the flow control system 888, e.g., the operation of any pumps housed by the flow control system. The control by the processing system 8889 may be according to any desired emulation or simulation technique. The processing system may for example be embodied by a workstation or other computer running software programs for providing input to the flow control system. Such systems may be completely standard and known in the art.

The system 800 may further include an optical inspection device 887 for observing 3D-cell clusters within one or more recesses 522 within the bottom of the chamber 520. In this case, and as most usual, the optical inspection device collects optical radiation 897 in the visual and/or UV light range using its objective. As such the beam must pass the perforated membrane of a recess 522 in addition to part of the cover plate 544. Hence, all of these preferably are at least to some extent transparent to the optical radiation involved. The silicone elastomer substrate materials are in this respect preferred material. Microscope systems as known in the art may be used in system 800.

The thickness of any cover plate in the Z-direction (perpendicular to the bottom) and at the location of the chamber 522 are preferably chosen such that a good quality focus at a desired magnification can be obtained using the systems attainable focal distances with its light objectives.

In summary there is disclosed is a device for culturing a three-dimensional cell cluster in at least one recess having a recess depth of at least substantially 50 µm, an effective recess diameter of at least substantially 50 µm, and a recess depth of preferably at least substantially half of the recess depth. Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. If the term "adapted to" is used in the claims or description, it is noted the term "adapted to" is intended to be equivalent to the term "configured to". As used herein, the term "one or more chosen from the group consisting of A and B" can mean: "one, or more, from A", or "one, or more, from B", or "one, or more, from A and one, or more, from B". Thus, there can be only of A, or only one of B, or a composition of multiple of A, or a composition of multiple of B, or a composition of at least one of A and at least one of B. Any reference signs in the claims should not be construed as limiting the scope.

## Claims

**1.** A device for culturing a three-dimensional cell cluster, the device comprising:
a substrate comprising a first substrate surface having at least one recess therein, the at least one recess being configured to hold the three-dimensional cell cluster at least partially therein, the at least one recess having a recess bottom and a recess depth of at least substantially 50 µm measured from the first substrate surface to the recess bottom, the at least one recess having a recess opening extending within the first substrate surface, the recess opening having an effective recess diameter of at least substantially 50 µm.

**2.** The device of claim 1, wherein the recess opening area is at most substantially 20 mm².

**3.** The device of claim 1 or 2, wherein the recess depth is at least substantially half of the effective recess diameter.

**4.** The device of any one of the previous claims, wherein the recess has a circularly shaped cross-section perpendicular to the direction of recess depth measurement.
More preferably wherein the recess has a shape in the form of a cylinder or frustum of a cone the larger base of which forms the recess opening.

**5.** The device of any one of the previous claims with the proviso that the device does not comprise the device wherein the substrate is an integrally formed substrate comprising a chamber comprising the at least one recess and wherein the integrally formed substrate comprises a filter chamber housing a filter membrane that divides the filter chamber in a first sub-chamber and a second sub-chamber, wherein the filter membrane is not part of a bottom of a recess for cultivating 3D-cell cultures.

**6.** The device of any one of the previous claims, wherein the recess bottom is permeable for fluid. Preferably, the permeability is implemented by a plurality of pores each of which having a pore diameter of at most substantially 20 µm. Preferably the pore diameter is at most 10 µm. The pore diameter can be in the range of 2 µm to 8 µm .

**7.** The device of any one of previous claims, wherein the bottom of the at least one recess comprises a membrane having a membrane thickness of at most 100 µm.

**8.** The device of claim 6 and 7, wherein the membrane comprises the pores.

**9.** The device of any one of the previous claims, wherein the at least one recess comprises a plurality of recesses spaced apart across the surface.

**10.** The device of any one of the previous claims, wherein the substrate comprises an elastomeric material and the at least one recess is formed in the elastomeric material. Preferably the membrane is also formed in the elastomeric material. Preferably the elastomeric material is transparent for UV and/or visible light. Preferably the elastomeric material is a silicone-based elastomeric material.

**11.** The device of any one of the previous claims, wherein the device comprises at least one chamber for holding a fluidic medium, the at least one chamber having a chamber bottom, the chamber bottom comprising the first substrate surface.

**12.** The device of claims 10 and 11, the device further comprising a body made of a stiff material, the body comprising the at least one chamber.

**14.** The device of any one of the claims 10 to 12, wherein the at least one chamber comprises a plurality of chambers.

**15.** The device of any one of the previous claims, the device comprising at least one fluid channel arranged to transport a fluidic medium from or to the at least one recess.

**16.** A method of cultivating a 3D-cell cluster in the at least one recess of a device as claimed in any of the claims 1 to 15 by adding cells and a fluid medium to the at least one recess.

**17.** A method comprising subjecting a 3D-cell cluster in the at least one recess of a device as claimed in any of the claims 1 to 15 to a drug.

**18.** Use of a device as claimed in any one of the claims 1 to 15 for growing, cultivating or investigating spheroids or organoids.
